# EUROPÄISCHE PATENTSCHRIFT

(11) **EP 2 919 923 B1**
(45) Veröffentlichungstag und Bekanntmachung des Hinweises auf die Patenterteilung: **22.06.2022**
(21) Anmeldenummer: 13789545.4
(22) Anmeldetag: 11.11.2013
(51) Int. Cl.: B08B 3/02, B08B 3/04, B08B 13/00, B08B 9/032, B63C 11/02

(54) **HALTERUNGS-SORTIMENT UND REINIGUNGSGERÄT ZUR REINIGUNG VON ATEMGERÄTEN**
HOLDER PRODUCT RANGE AND CLEANING APPARATUS FOR CLEANING BREATHING APPARATUSES
ASSORTIMENT À ÉLÉMENTS DE RETENUE ET APPAREIL DE NETTOYAGE DESTINÉ À NETTOYER LES VOIES RESPIRATOIRES

(30) Priorität: 13.11.2012 DE 102012220646
(43) Veröffentlichungstag der Anmeldung: 23.09.2015
(73) Patentinhaber: MEIKO Maschinenbau GmbH & Co. KG, 77652 Offenburg (DE)
(72) Erfinder: FALK, Herbert, 77654 Offenburg (DE); WÖRNER, Heiko, 77815 Bühl (DE)
(74) Vertreter: Altmann Stößel Dick Patentanwälte PartG mbB
(86) Internationale Anmeldenummer: PCT/EP2013/073474
(87) Internationale Veröffentlichungsnummer: WO 2014/076026

(56) Entgegenhaltungen:
- DE-A1- 10 020 835
- DE-A1-102010 029 221
- DE-U1- 20 003 744

## Beschreibung

### Gebiet der Erfindung

Die Erfindung betrifft ein Sortiment von Halterungen für Atemgeräte sowie ein Reinigungsgerät und ein Verfahren zur Reinigung von Atemgeräten. Derartige Sortimente, Reinigungsvorrichtungen und Verfahren werden allgemein zur Reinigung und optional auch zusätzlich zur Hygienisierung und/oder Desinfektion von Atemgeräten oder auch Bestandteilen derselben eingesetzt. Allgemein können die im Rahmen der vorliegenden Erfindung beschriebenen Vorrichtungen und Verfahren eingesetzt werden, um beispielsweise Atemgeräte für Rettungskräfte wie Feuerwehr, technische Hilfsorganisationen oder Rettungssanitäter zu reinigen. Alternativ oder zusätzlich können auch Atemgeräte für Taucher oder allgemein Personen in lebensfeindlichen oder kritischen Arbeitsumgebungen gereinigt werden. Somit sind die Vorrichtungen und Verfahren gemäß der vorliegenden Erfindung beispielsweise auch im Rahmen des Einsatzes von Streitkräften und Sicherheitskräften wie beispielsweise Polizisten einsetzbar. Auch für Atemgeräte im medizinischen Bereich, beispielsweise Atemmasken für Sauerstoffversorgung und Operationen, lassen sich die vorgeschlagenen Vorrichtungen und Verfahren grundsätzlich einsetzen. Insbesondere kommt allgemein die Reinigung von Atemmasken oder Lungenautomaten im Einsatzgebiet in Betracht.

### Stand der Technik

Atemgeräte wie beispielsweise Atemmasken, insbesondere Atemschutzmasken, oder Lungenautomaten, sind in der Regel Bestandteil der persönlichen Schutzausrüstung beispielsweise von Rettungskräften, Streitkräften oder Sicherheitskräften. So sind aus dem Stand der Technik eine Vielzahl von Atemgeräten für unterschiedliche Einsatzzwecke bekannt. Beispielsweise verwenden Rettungskräfte wie Feuerwehren Atemschutzmasken mit Filtern zur Entfernung schädlicher Bestandteile aus der angesaugten Atemluft. In vielen Fällen wird jedoch, alternativ oder zusätzlich zu einem Filter, ein so genannter Lungenautomat verwendet, über welchen der Benutzer mit einem Atemgas, beispielsweise Pressluft, beatmet werden kann. Lungenautomaten, welche häufig auch als Atemregler bezeichnet werden, ermöglichen es allgemein einem Benutzer, aus einer Druckgasflasche oder einem anderen Druckgasanschluss zu atmen und sich auf diese Weise beispielsweise unter Wasser oder in einer anderen, nicht atembaren oder giftigen Atmosphäre aufzuhalten. Dazu wird das Druckgas, beispielsweise komprimierte Luft, aus dem Druckgasanschluss durch den Lungenautomaten auf einen in einer Arbeitsumgebung des Benutzers herrschenden Druck angepasst.

Die Atemgeräte oder Bestandteile derselben müssen in der Regel nach jedem Einsatz gereinigt, hygienisiert, getrocknet, geprüft und gegebenenfalls instandgesetzt und verpackt werden. Mit der Reinigung sollen alle Verschmutzungen infolge eines Gebrauchs oder einer Lagerung entfernt werden, so dass die Atemgeräte makroskopisch sauber und hygienisch einwandfrei bereitgestellt werden können, beispielsweise für die nächsten Schritte einer Aufbereitung. Dieselben Anforderungen gelten in der Regel auch für andere Bestandteile von Atemgeräten, wie beispielsweise Anbau- und Zubehörteile von Atemmasken, wie beispielsweise Filter oder Lungenautomaten. Da Atemgeräte bzw. deren Bestandteile in der Regel sicherheitsrelevante Vorrichtungen sind, sind bei der Reinigung dieser Vorrichtungen mehrere Anforderungen zu beachten. Neben einer ausreichenden Reinigung und Hygienisierung ist beispielsweise in vielen Fällen zu beachten, dass Zubehörteile aus technischen Gründen den jeweiligen Atemmasken zugeordnet bleiben müssen. Weiterhin besteht in der Regel die Anforderung, dass gasführende Bereiche bestimmter Elemente von Atemgeräten, beispielsweise gasführende Bereiche von Lungenautomaten, nicht mit Reinigungsfluid, beispielsweise nicht mit Wasser und/oder Reinigungslösung, in Berührung kommen dürfen.

In vielen Fällen werden Atemgeräte, wie beispielsweise Atemmasken und deren Zubehör, entweder von Hand gereinigt oder in modifizierten Wäschewaschmaschinen mit Hilfe von Schutzbeuteln und/oder mit Hilfe von Adaptern gewaschen. Die Reinigung von empfindlichen Bauelementen der Atemgeräte, wie beispielsweise Lungenautomaten, erfolgt in vielen Fällen manuell. Gegebenenfalls kann eine manuelle Reinigung durch ein Einlegen in Ultraschall-Reinigungsgeräte unterstützt werden.

Die meisten bekannten Verfahren und Vorrichtungen zur Reinigung von Atemgeräten wie beispielsweise Atemmasken, insbesondere Atemschutzmasken, und deren Zubehörteilen weisen eine Vielzahl von Nachteilen auf. So ist eine manuelle Reinigung der Atemgeräte sehr arbeitsintensiv. Zudem ist der Reinigungsprozess in diesem Fall stark von der einzelnen Reinigungskraft beeinflusst und lässt sich hierdurch kaum standardisieren.

Aus EP 0 935 687 B1 ist allgemein eine Waschmaschine bekannt, welche einen Laugenbehälter mit einer Trommel aufweist. Ein Mantel der Trommel weist eine zum Trommelinneren gerichtete Wölbestruktur auf, wobei auf zum Trommeläußeren gerichteten Randkonturen der Wölbung in ihren Eckpunkten Löcher angeordnet sind. Mit derartigen Waschmaschinen sind grundsätzlich besonders schonende Reinigungen von Ausrüstungsgegenständen für Rettungskräfte möglich.

Die Reinigung in modifizierten Wäschewaschmaschinen ist jedoch in der Praxis ebenfalls vergleichsweise zeitintensiv. Zudem sind die Atemgeräte oder deren Bestandteile in vielen Fällen nach der Reinigung im Inneren mit Reinigungsfluid befüllt, da diese entweder in der Trommel der Waschmaschine wahllos angeordnet sind oder da deren Stellung innerhalb der Trommel fixiert ist und nicht gezielt beeinflusst werden kann, so dass die Atemgeräte mit Beendigung der Reinigungsprozedur in der Regel nicht leerlaufen können. Zubehörteile können meist nicht in der Waschmaschine mitgereinigt werden, da während des Prozesses häufig eine individuelle Zuordnung verloren geht.

Aus dem medizinischen Umfeld sind weiterhin Spülmaschinen bekannt, in welchen Beatmungsschläuche gespült werden können. Derartige Spülmaschinen sind in der Regel als Einkreis-Maschinen, auch als Wasserwechsel-Maschinen bezeichnet, ausgestaltet. Dies bedeutet, dass die Spülmaschinen einen Spülbehälter aufweisen, in welchem die Reinigung und die Konditionierung der Reinigungsflüssigkeit erfolgen. Für den Wechsel einer Reinigungsflüssigkeit, beispielsweise für einen Wechsel von einer Waschlauge hin zu einer Klarspülflüssigkeit, ist ein vollständiger Wasserwechsel innerhalb des Behälters erforderlich.

Besonders kritisch ist mit den bekannten Verfahren und Vorrichtungen die Reinigung von gasführenden Elementen der Atemgeräte wie beispielsweise einem oder mehreren Rohrleitungen, Schläuchen oder Ventilen und/oder Lungenautomaten. Für derartige Lungenautomaten stehen bislang nur wenige automatisierte Reinigungsverfahren zur Verfügung, welche den oben genannten Sicherheitsanforderungen genügen würden. Das aus DE 10 2007 012 768 B4 bekannte Reinigungsverfahren weist hingegen die oben beschriebenen Nachteile auf und ist vergleichsweise aufwändig. Weiterhin ermöglicht die dargestellte Vorrichtung nicht gleichzeitig die Reinigung und ggf. Desinfektion von Lungenautomaten und Atemschutzmasken sowie Zubehörteilen. Auch Spülmaschinen aus dem medizinischen Umfeld, welche für eine Maskenreinigung adaptiert wurden, sind nicht dafür geeignet, Lungenautomaten zu reinigen und ausreichend zu hygienisieren.

Aus EP 1 088 928 A1 ist ein Haltesystem für Atemschutzmasken in einer Wäschebehandlungsmaschine bekannt. Das Haltesystem weist einen Tragbügel auf, welcher in einer Trommel der Wäschebehandlungsmaschine mitdrehend angeordnet ist und mit welchem die Atemschutzmasken verbunden werden können.

Aus DE 200 03 743 U1 und aus DE 298 22 172 U1 sind jeweils Vorrichtungen zum Behandeln von Schutzanzügen bekannt. Dabei werden Kleiderbügel verwendet, welche flexible Luftaustrittsdüsen umfassen. Die Kleiderbügel sind jeweils an einer Schwenkvorrichtung befestigt. Eine Reinigung von Atemgeräten ist allgemein mittels der gezeigten Vorrichtungen nicht oder nur schwer möglich.

In DE 11 74 169 B wird eine Vorrichtung zur Reinigung von Atemschutzmasken offenbart. Dabei ist in einem Gehäuse ein drehbar gelagertes Rohrgestell vorgesehen, bei welchem die zu reinigenden Masken auf Halterungen aufgespannt werden. Mittels des Drehgestells werden die Atemschutzmasken mit einer Drehbewegung durch ein Bad einer Reinigungsflüssigkeit in einer Wanne hindurch bewegt.

Aus DE 10 2007 012 768 B4 sind jedoch ein Verfahren und eine Vorrichtung zur Reinigung von Lungenautomaten bekannt. Dabei werden die zu reinigenden Gegenstände auf Halterungen eines rotierenden Elements aufgesteckt und mehrmals in ein Flüssigkeitsbad mit Reinigungsflüssigkeit, Desinfektionsflüssigkeit und Spülflüssigkeit eingetaucht. Lungenautomaten werden dabei zuerst zur Dichtsetzung zwischen Ventil und Schlauchanschluss mit Druckluft beaufschlagt und danach in das Flüssigkeitsbad eingetaucht. Nachteilig an derartigen Tauchverfahren ist jedoch, dass aufwändige Halterungen mit entsprechenden Aktoren erforderlich sind, um durch entsprechende Bewegungen eine Entfernung von Reinigungsfluid aus den verschiedenen Hohlräumen nach der Reinigung zu gewährleisten.

In US 3,881,503 A wird eine Vorrichtung zum Waschen und Dekontaminieren von Anästhesie-Ausrüstungsgegenständen beschrieben. Dabei ist unter anderem ein Düsensystem vorgesehen, durch welches unter hohem Druck Wasserstrahlen auf das Reinigungsgut aufgesprüht werden können.

Aus DE 100 20 835 A1 ist eine Vorrichtung zur Behandlung von Atemschutzmasken bekannt. In dieser ist eine Aufnahme zur Bestückung mit Atemschutzmasken vorgesehen, bei welcher eine Kopplung der Atemschutzmasken mittels des Atemgeräteanschlusses an die Aufnahme erfolgt. Weiterhin wird beschrieben, dass in einer Kabine der Vorrichtung eine Sammeleinrichtung zur Sammlung von Behandlungsmitteln sowie eine Pumpe für die Zufuhr der Behandlungsmittel vorgesehen sind.

DE 2 131 055 beschreibt eine Waschvorrichtung für hohle Gegenstände, mit einem Bottich und einer in den Bottich eingeführten Antriebswelle. Weiterhin ist eine Halteeinrichtung vorgesehen, mit deren Hilfe von der Welle aus mehrere zu waschende hohle Gegenstände bewegt werden können. Weiterhin ist vorgesehen, dass unterschiedliche Arten von Körben (beispielsweise ein Narkose-Korb 42 oder ein Inhalations-Korb 195) in den Waschraum eingebracht werden können.

Aus DE 10 2005 033 618 B3 ist eine Vorrichtung zur Reinigung von Atemschutzmasken bekannt. Die Vorrichtung umfasst ein verschließbares Gehäuse sowie mindestens eine in einem Träger angeordnete Aufnahme für mindestens eine Atemschutzmaske. Weiterhin ist eine Düsenanordnung und eine Bürstenanordnung vorgesehen, wobei durch eine Bewegung der Atemschutzmasken eine Bebürstung der Atemschutzmasken erfolgt. Mittels der offenbarten Vorrichtung ist allerdings eine individuelle Zuordnung und Reinigung von Zubehörteilen von Masken nicht möglich. Weiterhin ist die Reinigung von gasführenden Elementen, wie beispielsweise Lungenautomaten, mit der offenbarten Vorrichtung nicht möglich. Auch aus DE 200 03 744 U1 ist eine Vorrichtung zum Reinigen, Desinfizieren und Trocknen von Atemschutzmasken bekannt, welche ein Tragegestell mit einem zugeordneten Düsensystem und einzelnen Behandlungsplätzen aufweist. Auch diese Vorrichtung ist grundsätzlich nicht zur Reinigung von gasführenden Elementen und Zubehörteilen geeignet.

Aus DE 10 2007 009 936 A1 ist eine Reinigungsvorrichtung für Pressluftatmer bekannt. Diese weist einen durch ein Schutzgitter begrenzten Aufnahmeraum sowie rotierende Düsenträger auf. Die Düsenträger befinden sich dabei außerhalb des Schutzgitters. Nachteilig an der dargestellten Vorrichtung ist jedoch, dass Reinigungsflüssigkeit in gasführende Bereiche eindringen kann.

Aus WO 2011/144518A2 ist eine Reinigungsvorrichtung zur Reinigung von Atemgeräten bekannt, insbesondere von Lungenautomaten und/oder Atemmasken. Die Reinigungsvorrichtung umfasst mindestens eine Reinigungskammer zur Aufnahme mindestens eines Atemgeräts. Die Reinigungsvorrichtung weist weiterhin mindestens eine Fluideinrichtung zur Beaufschlagung des Atemgeräts mit mindestens einem Reinigungsfluid auf. Die Reinigungsvorrichtung weist weiterhin mindestens eine Druckbeaufschlagungsvorrichtung mit mindestens einem Druckanschluss auf. Der Druckanschluss ist mit mindestens einem gasführenden Element des Atemgeräts verbindbar. Die Druckbeaufschlagungsvorrichtung ist eingerichtet, um das gasführende Element mit Druckgas zu beaufschlagen. Weiterhin wird eine Halterung zur Aufnahme von Atemgeräten beschrieben, welche insbesondere derart ausgestaltet sein kann, dass Zubehör der Atemgeräte jeweils einem Atemgerät zugeordnet bleibt. Die Halterung kann ein korbartiges Behältnis umfassen, in welchem das Zubehör aufgenommen wird.

Trotz der mit den bekannten Verfahren und Vorrichtungen erzielten Vorteile, insbesondere im Rahmen einer automatisierten Reinigung von Atemgeräten, besteht nach wie vor in der Praxis ein erheblicher Optimierungsbedarf. Insbesondere hinsichtlich der bekannten Halterungen, welche das Atemgerät oder Teile desselben während des Reinigungsvorgangs aufnehmen, ist in vielen Fällen festzustellen, dass diese Halterungen vergleichsweise unspezifisch für das jeweils verwendete Atemgerät sind. So werden in der Praxis in vielen Fällen Universal-Halterungen eingesetzt, beispielsweise in Form von Körben oder anderen Ladungsträgern, in welchen die zahlreichen Bestandteile des Atemgeräts in vielen Fällen ungeordnet und ungerichtet aufgenommen sind. Dementsprechend kann ein Reinigungs- und Desinfektionsergebnis in vielen Fällen nicht optimal und stark schwankend sein.

### Aufgabe der Erfindung

Es ist dementsprechend eine Aufgabe der vorliegenden Erfindung, Vorrichtungen und Verfahren zur Reinigung von Atemgeräten bereitzustellen, welche die Nachteile bekannter Vorrichtungen und Verfahren zumindest weitgehend vermeiden. Insbesondere soll eine zuverlässige Reinigung und, optional, eine zuverlässige Hygienisierung oder sogar Desinfektion der verschiedenen, auf unterschiedliche Weise geformten Bestandteile der Atemgeräte ermöglicht werden, welche eine hohe Reproduzierbarkeit hinsichtlich des Ergebnisses aufweist.

### Offenbarung der Erfindung

Diese Aufgabe wird gelöst durch die Vorrichtungen und das Verfahren mit den Merkmalen der unabhängigen Ansprüche. Vorteilhafte Weiterbildungen der Erfindung, welche einzeln oder in Kombination realisierbar sind, sind in den abhängigen Ansprüchen dargestellt.

Hier und im Folgenden werden die Begriffe "haben", "aufweisen", "einschließen", "umfassen" oder "beinhalten" sowie beliebige grammatikalische Variationen dieser Begriffe in nicht-abschließender Weise verwendet. Dementsprechend können sich diese Begriffe sowohl auf eine Situation beziehen, in welcher, abgesehen von den Merkmalen, welche durch diese Begriffe eingeleitet werden, keine weiteren Merkmale vorgesehen sind, als auch auf die Situation, in welcher ein oder mehrere weitere Merkmale vorhanden sind. Beispielsweise kann sich die Aussage "A weist B auf" sowohl auf die Situation beziehen, in welcher A neben dem Merkmal B keine weiteren Merkmale aufweist, als auch auf die Situation, in welcher A, neben dem Merkmal B, ein oder mehrere weitere Merkmale umfasst, beispielsweise ein Merkmal C, Merkmale C und D oder sogar weitere Merkmale.

In einem ersten Aspekt der vorliegenden Erfindung wird ein Sortiment zur Reinigung von Atemgeräten vorgeschlagen, welches mindestens zwei Halterungen aufweist. Unter einem Sortiment, häufig auch bezeichnet als Kit, wird allgemein im Rahmen der vorliegenden Erfindung eine Mehrzahl von Objekten verstanden, welche ganz oder teilweise unabhängig voneinander gehandhabt werden können und welche einander in ihren Funktionen unterstützen oder ergänzen können.

Das Sortiment dient zur Reinigung und optional zusätzlich auch zur Desinfektion von Atemgeräten. Unter Atemgeräten werden dabei allgemein Vorrichtungen verstanden, welche in irgendeiner Weise zur Bereitstellung von Atemgas an mindestens einen menschlichen und/oder tierischen Benutzer eingerichtet sind. Für mögliche Einsatzzwecke kann beispielsweise auf die obige Beschreibung verwiesen werden. Bei diesen Atemgeräten kann es sich dabei um vollständige, betriebsfertige Atemgeräte handeln, oder auch um Bestandteile derselben, so dass im Folgenden zwischen Atemgeräten und deren Bestandteilen begrifflich nicht unterschieden wird. Insbesondere können die Atemgeräte, wie nachfolgend näher ausgeführt wird, eine oder mehrere Atemmasken, insbesondere Atemschutzmasken, einen oder mehrere Schläuche, ein oder mehrere Ventile, einen oder mehrere Filter, einen oder mehrere Druckgasbehälter, einen oder mehrere Lungenautomaten, eine oder mehrere Druckgasflaschen, ein oder mehrere Tragegestelle für Druckgasflaschen oder auch grundsätzlich beliebige Kombinationen der genannten und/oder anderer Elemente umfassen.

Das Sortiment umfasst eine Mehrzahl von Halterungen. Dabei sind mindestens zwei Halterungen vorgesehen, nämlich mindestens eine erste Halterung und mindestens eine zweite Halterung, deren Einsatzzwecke und Ausgestaltungen im Folgenden näher beschrieben werden. Daneben können eine oder mehrere weitere Halterungen vorgesehen sein, welche ebenfalls im Nachfolgenden näher beschrieben werden.

Das Sortiment und insbesondere die Halterungen desselben umfassen mindestens eine erste Halterung zur Reinigung von Atemmasken und Lungenautomaten. Diese erste Halterung umfasst mindestens eine Masken-Haltevorrichtung zur Positionierung mindestens einer Atemmaske. Allgemein wird dabei im Rahmen der vorliegenden Erfindung unter einer Positionierung eine gerichtete Halterung eines Gegenstands verstanden, so dass der Gegenstand nach Positionierung eine definierte Position und/oder eine definierte räumliche Ausrichtung aufweist, zumindest im Rahmen vorgegebener Toleranzen.

Die erste Halterung weist weiterhin mindestens eine Lungenautomat-Haltevorrichtung zur Positionierung mindestens eines Lungenautomaten und mindestens eine Druckbeaufschlagungsvorrichtung mit mindestens einem Druckanschluss auf. Der Druckanschluss ist mit dem Lungenautomaten verbindbar, und die Druckbeaufschlagungsvorrichtung ist eingerichtet, um den Lungenautomaten während einer Reinigung mit Druckgas zu beaufschlagen.

Das Sortiment und insbesondere die Halterungen desselben umfassen weiterhin mindestens eine zweite Halterung zur Reinigung von Traggestellen für Druckgasflaschen für Atemgeräte. Allgemein ist dabei unter einem Traggestell ein Gestell zu verstehen, welches auf den Rücken eines Benutzers aufgesetzt werden kann und mittels eines oder mehrerer Tragriemen, welche Bestandteil des Traggestells selbst sein können oder auch separate Bauteile sein können, am Rücken des Benutzers fixierbar ist. Die Tragriemen können beispielsweise einen oder mehrere Schultergurte umfassen und/oder einen oder mehrere Beckengurte. Alternativ oder zusätzlich zu einem oder mehreren Tragriemen können die Tragriemen auch in Form einer Weste ausgestaltet sein, mittels derer das Traggestell auf dem Rücken des Benutzers fixierbar ist. Die Druckgasflaschen können beispielsweise Druckgasflaschen mit Atemluft und/oder einem Sauerstoff-Gemisch umfassen. Die zweite Halterung umfasst mindestens eine Traggestell-Haltevorrichtung zur Positionierung mindestens eines Traggestells.

Das Sortiment ist dabei allgemein derart ausgestaltet, dass die Halterungen derart dimensioniert sind, dass diese austauschbar in eine Reinigungsvorrichtung zur Reinigung des Atemgeräts einbringbar sind. So können wahlweise die erste Halterung und/oder die zweite Halterung verwendet werden und/oder optional mindestens eine weitere Halterung. Diese Dimensionierung kann beispielsweise derart erfolgen, dass die Halterungen, beispielsweise die erste Halterung und die zweite Halterung sowie optional mindestens eine weitere Halterung, eine zumindest im Rahmen von Bauteiltoleranzen identische Grundfläche und/oder identische Außenabmessungen aufweisen. Wie unten noch näher ausgeführt wird, kann dies insbesondere dadurch erfolgen, dass die erste Halterung und die zweite Halterung, sowie gegebenenfalls optional mögliche weitere Halterungen, einen baugleichen und/oder identischen Grundkorb aufweisen können, auf welchen jeweils unterschiedliche Aufsätze aufgebracht oder aufbringbar sind.

Die Masken-Haltevorrichtung kann insbesondere ein Gestell umfassen. Dieses Gestell kann insbesondere mindestens einen Haltebügel für die mindestens eine Atemmaske aufweisen, wobei die Atemmaske auf den Haltebügel aufgestülpt werden kann. Durch dieses Aufstülpen kann beispielsweise der Haltebügel vollständig oder teilweise in einen Innenraum der Atemmaske eindringen. Der Haltebügel kann insbesondere derart eingerichtet sein, dass die Atemmaske derart auf den Haltebügel aufgestülpt werden kann, dass eine Innenseite der Atemmaske nach oben weist. Unter "oben" ist dabei eine Raumrichtung zu verstehen, welche in einem Zustand, in welchem die erste Halterung in eine Reinigungskammer der Reinigungsvorrichtung eingebracht ist, räumlich nach oben weist. Auf diese Weise kann insbesondere von oben her eine Beaufschlagung der Innenseite der Atemmaske, welche im Einsatz häufig stark durch Schweiß oder Speichel belastet wird, mit Reinigungsfluid erfolgen. Gleichzeitig sollte dabei die Innenseite derart positioniert sein, dass diese schräg zur Horizontalen steht, damit Reinigungsfluid aus dieser Innenseite der Atemmaske nach unten ablaufen kann. Die Masken-Haltevorrichtung kann entsprechend ausgestaltet sein.

Das Gestell der Masken-Haltevorrichtung kann insbesondere ein Drahtgestell sein. Der Haltebügel kann insbesondere einen Drahtbügel umfassen, insbesondere einen U-förmig gebogenen Drahtbügel.

Das Gestell kann insbesondere weiterhin mindestens einen weiteren Haltebügel für mindestens eine Innenmaske der Atemmaske aufweisen. So ist bei vielen Atemmasken zusätzlich innerhalb einer äußeren Maske eine Innenmaske vorgesehen, welche sich beispielsweise eng über Nase und/oder Mund des Benutzers legen kann. Diese Innenmaske hat typischerweise im unteren Bereich eine Öffnung, welche beispielsweise direkt mit einer Ausatem-Öffnung der Atemmaske verbunden sein kann. Durch diese Ausführung kann die Atemmaske, insbesondere die Atemschutzmaske, in einen inneren und einen äußeren Bereich aufgeteilt sein. An ihren Seitenbereichen weist die Innenmaske typischerweise ein oder mehrere Rückschlagventile auf, die einen Luftstrom ins Innere zulassen. Hierdurch kann ein Frischluftstrom von der Druckgasflasche, beispielsweise der Druckluftflasche, über einen Lungenautomaten in den äußeren Bereich der Atemmaske und über die genannten Rückschlagventile in die Innenmaske erfolgen. Durch diese Konstruktion kann verhindert werden, dass feuchte, ausgeatmete Luft in den äußeren Bereich der Atemmaske eindringt, und es kann beispielsweise ein Beschlagen einer oder mehrerer Sichtscheiben der Atemmaske verhindert werden. Durch das Vorsehen mindestens eines Haltebügels für mindestens eine Innenmaske in der ersten Halterung kann dieser Tatsache Rechnung getragen werden.

Auch der mindestens eine weitere Haltebügel für die mindestens eine Innenmaske kann beispielsweise als Drahtbügel ausgestaltet werden.

Die Masken-Haltevorrichtung kann optional ein oder mehrere Fixierelemente zum Fixieren der mindestens einen Atemmaske und/oder der mindestens einen optionalen Innenmaske aufweisen. Diese Fixierelemente können beispielsweise jeweils eine geöffnete Position und eine geschlossene Position aufweisen. In der geöffneten Position kann beispielsweise ein Einbringen der jeweiligen Atemmaske und/oder optionalen Innenmaske in die Masken-Haltevorrichtung und/oder eine Entfernung der jeweiligen Atemmaske und/oder optionalen Innenmaske aus der Masken-Haltevorrichtung erfolgen. In der geschlossenen Position kann beispielsweise eine Fixierung der jeweiligen Atemmaske und/oder optionalen Innenmaske erfolgen, beispielsweise um ein Verrutschen während eines Reinigungsvorgangs zu verhindern. Das mindestens eine optionale Fixierelement kann beispielsweise beweglich gelagert sein. Beispielsweise kann dieses einen Drahtbügel und/oder Drahtring umfassen. Beispielsweise kann jeweils ein Fixierelement für eine Innenmaske beweglich auf einen Drahtbügel und/oder eine andere Halterung einer zugehörigen Atemmaske aufgeschoben sein. Auch andere Ausgestaltungen sind jedoch denkbar, beispielsweise schwenkbare und/oder auf andere Weise verschiebbare Ausführungsformen.

Weitere mögliche Ausgestaltungen betreffen die Lungenautomat-Haltevorrichtung. So kann diese Lungenautomat-Haltevorrichtung beispielsweise mindestens eine Aufnahme aufweisen, in welche ein Stutzen des Lungenautomaten einsteckbar ist und in welcher der Stutzen nach dem Einstecken fest gehaltert ist. Beispielsweise kann diese Aufnahme eine runde Öffnung umfassen. Die Aufnahme kann beispielsweise eine Dichtung umfassen, in welche der Stutzen des Lungenautomaten eingesteckt werden kann. So kann die Aufnahme beispielsweise mindestens eine Reibbremse zur reibschlüssigen Halterung des Lungenautomaten umfassen. Diese Reibbremse kann beispielsweise in Form mindestens eines Gummirings und/oder mindestens eines Dichtrings realisiert werden, in welche der Stutzen eingesteckt werden kann und reibschlüssig gehaltert werden kann.

Der oben genannte Druckanschluss kann insbesondere mindestens eine Kupplung zum Ankoppeln eines Druckgasschlauchs des Lungenautomaten aufweisen. Hierbei können beispielsweise standardisierte Kupplungen verwendet werden. Die Kupplung kann auch beispielsweise mindestens einen Adapter zum Ankoppeln mehrerer unterschiedlicher Arten von Druckgasschläuchen aufweisen.

Die Kupplung kann insbesondere durch mindestens eine abnehmbare Kappe verschließbar sein, wenn kein Druckgasschlauch angeschlossen ist. Beispielsweise kann die abnehmbare Kappe eine Metallkappe und/oder eine Kunststoffkappe, beispielsweise eine Elastomerkappe, sein. Die Kappe kann beispielsweise über einen flexiblen Sicherungssteg mit dem Druckanschluss verbunden sein, so dass ein Verlust dieser Kappe auch im abgenommenen Zustand verhindert wird.

Die Druckbeaufschlagungsvorrichtung kann beispielsweise mindestens eine Druckluftzufuhr aufweisen. Die Druckluftzufuhr kann insbesondere in eine Druckgasleitung münden, wobei der Druckanschluss auf die Druckgasleitung aufgesetzt sein kann. Die Druckluftzufuhr kann insbesondere mindestens einen Schlauch umfassen.

Die optionale Druckgasleitung kann insbesondere als tragendes Bauteil der ersten Halterung ausgestaltet sein und kann beispielsweise zumindest die Lungenautomat-Haltevorrichtung tragen. Als tragendes Bauteil wird dabei im Rahmen der vorliegenden Erfindung allgemein ein starres Bauteil bezeichnet, welches zumindest insoweit formstabil ist, dass dieses unter Belastung durch das Atemgerät oder Teile desselben seine Form zumindest makroskopisch nicht ändert.

So kann die Druckgasleitung insbesondere als Rahmen ausgestaltet sein. Dieser Rahmen kann beispielsweise ein rechteckiger Rahmen sein. Der Rahmen kann insbesondere ringförmig ausgestaltet sein, insbesondere ringförmig geschlossen sein. So kann dieser Rahmen beispielsweise ein Rohrrahmen sein, welcher ringförmig ausgestaltet ist, insbesondere ringförmig geschlossen ist und welcher beispielsweise einen rechteckigen Querschnitt aufweisen kann. Auf diese Weise kann beispielsweise innerhalb der Druckgasleitung ringförmig gleichmäßig ein Druck angelegt werden.

Die Ausgestaltung der Druckgasleitung als Rahmen ist besonders vorteilhaft im Zusammenhang mit einer Ausgestaltung, bei welcher sich mehrere Halterungen einen gemeinsamen Grundkorb teilen, auf welchen jeweils Halterungs-spezifische Aufsätze wahlweise aufgesetzt werden können. Wie unten noch näher ausgeführt wird, weist das Sortiment mehrere Halterungen auf wobei die Halterungen jeweils einen Grundkorb und jeweils mindestens einen Aufsatz aufweisen, wobei der Grundkorb der Halterungen baugleich ist und somit beispielsweise zwischen den Halterungen austauschbar ist. So kann beispielsweise derselbe Grundkorb wahlweise für die mindestens zwei verschiedenen Halterungen verwendbar sein.

Die Aufsätze der Halterungen können dann beispielsweise unterschiedliche ausgestaltet sein. So kann beispielsweise die erste Halterung einen ersten Aufsatz aufweisen, der auf den Grundkorb aufsetzbar ist, und die zweite Halterung kann einen zweiten Aufsatz aufweisen, der ebenfalls auf den Grundkorb aufsetzbar ist. Weitere Halterungen können entsprechend weitere Aufsätze aufweisen.

Beispielsweise kann der erste Aufsatz die oben genannte Druckbeaufschlagungsvorrichtung mit der mindestens einen Druckluftzufuhr aufweisen, welche in die mindestens eine Druckgasleitung mündet, wobei der Druckanschluss vorzugsweise auf die Druckgasleitung aufgesetzt ist. Die Druckgasleitung kann als tragendes Bauteil des ersten Aufsatzes ausgestaltet sein und kann insbesondere zumindest die Lungenautomat-Haltevorrichtung tragen. Die Druckgasleitung kann als Rahmen ausgestaltet sein, insbesondere als geschlossener Rahmen.

So kann der durch die Druckgasleitung gebildete Rahmen beispielsweise dem ersten Aufsatz die erforderliche Stabilität verleihen. So kann ein Benutzer bei einem Wechsel der Aufsätze beispielsweise den ersten Aufsatz bei dem Rahmen ergreifen und von dem Grundkorb abnehmen oder auf den Grundkorb aufsetzen. Der Rahmen kann also die Handhabbarkeit und die Stabilität des Sortiments erheblich verbessern. Der Rahmen kann optional auch einen oder mehrere Griffe aufweisen, um ein Abnehmen oder Aufsetzen des ersten Aufsatzes auf den Grundkorb zu erleichtern. Weiterhin kann der Rahmen derart ausgestaltet sein, dass sämtliche Bestandteile der ersten Halterung, mit Ausnahme des Grundkorbs, direkt oder indirekt mit dem Rahmen verbunden sind oder verbindbar sind, so dass beispielsweise durch ein Abnehmen des Rahmens von dem Grundkorb sämtliche Bestandteile des ersten Aufsatzes von dem Grundkorb abnehmbar sind bzw. dass durch ein Aufsetzen des Rahmens auf den Grundkorb sämtliche Bestandteile des ersten Aufsatzes auf den Grundkorb aufsetzbar sind. Die Druckbeaufschlagungsvorrichtung kann also mindestens einer Doppelfunktion dienen, bei welcher einerseits eine Druckbeaufschlagung gewährleistet wird und bei welcher andererseits die Stabilität und die Handhabbarkeit des Sortiments erheblich verbessert werden.

Die erste Halterung kann insbesondere eingerichtet sein, um eine Mehrzahl von Atemmasken und eine Mehrzahl von Lungenautomaten aufzunehmen und kann dementsprechend eine Mehrzahl von Masken-Haltevorrichtungen und eine Mehrzahl von Lungenautomat-Haltevorrichtungen sowie eine Mehrzahl von Druckanschlüssen aufweisen. Insbesondere können dabei die Masken-Haltevorrichtungen und die Lungenautomat-Haltevorrichtungen, betrachtet entlang eines Umfangs der ersten Halterung, alternierend angeordnet sein. Diese alternierende Anordnung kann insbesondere eine erleichterte Zuordnung zwischen den Atemmasken und den Lungenautomaten ermöglichen, so dass ein Untermischungsschutz zumindest weitgehend gewährleistet werden kann.

Die erste Halterung weist weiterhin mindestens einen Kleinteilkorb auf. Unter einem Kleinteilkorb wird dabei allgemein im Rahmen der vorliegenden Erfindung eine Vorrichtung mit mindestens einem Innenraum zur Aufnahme von Kleinteilen der Atemgeräte verstanden, wobei der Innenraum zumindest weitgehend verschlossen ist. Dieser Kleinteilkorb kann beispielsweise quaderförmig ausgestaltet sein. Beispielsweise kann der Kleinteilkorb an seinem oberen Ende eine Öffnung aufweisen, durch welche die Kleinteile in den Innenraum des Kleinteilkorbs eingebracht werden können. Diese Öffnung kann beispielsweise durch einen oder mehrere Deckel verschlossen oder verschließbar sein.

Der Kleinteilkorb kann insbesondere eine Mehrzahl von Öffnungen aufweisen, also Öffnungen in mindestens einer Wand des Kleinteilkorbs, vorzugsweise in sämtlichen Wänden des Kleinteilkorbs, welche einen Innenraum des Kleinteilkorbs umschließen. Durch diese Öffnungen kann insbesondere Reinigungsfluid in den Innenraum des Reinigungskorbs eindringen. Diese Öffnungen können insbesondere einen Äquivalentdurchmesser von 3 mm bis 20 mm, insbesondere von 5 mm bis 15 mm und besonders bevorzugt von 10 mm aufweisen. Diese Öffnungsweiten, beispielsweise Maschenweiten, haben sich als besonders vorteilhaft für die Reinigung von Zubehörteilen von Atemgeräten herausgestellt, da auf diese Weise zum einen ausreichend Reinigungsfluid in den Innenraum des Kleinteilkorbs eindringen kann, um die Kleinteile ausreichend zu reinigen, und da andererseits die Kleinteile durch die Öffnungen nicht aus dem Innenraum herausfallen können.

Der Kleinteilkorb kann insbesondere ganz oder teilweise aus einem Drahtgeflecht hergestellt sein. Alternativ oder zusätzlich sind jedoch auch andere Materialien einsetzbar, beispielsweise Kunststoffmaterialien, beispielsweise ein Kunststoff-Maschengeflecht. Auch ein metallischer Kleinteilkorb, beispielsweise ein Blechkorb, ist grundsätzlich denkbar.

Der Kleinteilkorb kann beispielsweise von der ersten Halterung abnehmbar sein. So kann die erste Halterung beispielsweise mindestens eine Aufnahme zum Einsetzen des Kleinteilkorbs aufweisen. Beispielsweise kann die erste Halterung ein Drahtgestell aufweisen, in welchem eine oder mehrere Öffnungen für den mindestens einen Kleinteilkorb vorgesehen sind.

Besonders bevorzugt ist es, wenn eine Mehrzahl von Kleinteilkörben vorgesehen sind. Die Kleinteilkörbe können insbesondere durch einen gemeinsamen Deckel verschließbar sein. Die erste Halterung kann beispielsweise zur Reinigung einer Mehrzahl von Atemmasken und einer Mehrzahl von Lungenautomaten eingerichtet sein, wobei jedem Paar, bestehend aus einer Atemmaske und einem Lungenautomaten, jeweils ein Kleinteilkorb zugeordnet sein kann. Beispielsweise kann die oben beschriebene Aufnahme zum Einsetzen der Kleinteilkörbe derart ausgestaltet sein, dass stets ein bestimmter Kleinteilkorb einem Paar, bestehend aus einer Atemmaske und einem Lungenautomaten, zuweist. Auf diese Weise kann wiederum ein Untermischungsschutz gewährleistet werden.

Weitere mögliche Ausgestaltungen betreffen die Traggestell-Haltevorrichtung. So kann die Traggestell-Haltevorrichtung beispielsweise mindestens eine nach oben von der zweiten Halterung ragende Stütze aufweisen. Diese Stütze kann insbesondere im Wesentlichen senkrecht nach oben ragen. Dabei sind auch Abweichungen von einer vollständig senkrechten Orientierung denkbar, beispielsweise um nicht mehr als 20°, insbesondere um nicht mehr als 10°. Die Stütze kann insbesondere eine ebene Platte umfassen. Die ebene Platte kann ebenfalls beispielsweise eine Vielzahl von Öffnungen aufweisen. Durch diese Öffnungen kann beispielsweise gewährleistet werden, dass Reinigungsfluid von einer Rückseite der Platte, welche von dem Traggestell weg weist, hin zum Traggestell gelangen kann. Beispielsweise kann diese Platte aus einem metallischen Material und/oder aus einem Kunststoffmaterial hergestellt sein. So kann beispielsweise die Platte zumindest teilweise aus einem Drahtgeflecht hergestellt sein. Alternativ oder zusätzlich sind wiederum andere Alternativen denkbar, beispielsweise ein Kunststoffgeflecht mit einer Mehrzahl von Öffnungen und/oder eine perforierte Metallblech-Platte mit einer Mehrzahl von Öffnungen. Allgemein ist es wiederum besonders bevorzugt, wenn die Öffnungen einen Äquivalentdurchmessser von 3 mm bis 20 mm, vorzugsweise einen Äquivalentdurchmesser von 5 mm bis 15 mm und besonders bevorzugt einen Äquivalentdurchmesser von 10 mm aufweisen.

Die zweite Halterung kann weiterhin insbesondere mindestens einen Rahmen aufweisen. Die Stütze kann durch eine Mehrzahl von Querstreben mit diesem Rahmen verbunden und stabilisiert sein. So kann beispielsweise der Rahmen während einer Reinigung mindestens eines Traggestells im Wesentlichen horizontal innerhalb einer Reinigungskammer einer Reinigungsvorrichtung aufgenommen sein, während die Stütze im Wesentlichen vertikal zu diesem Rahmen angeordnet ist und durch die Mehrzahl von Querstreben mit dem Rahmen verbunden und stabilisiert ist. Auf diese Weise kann beispielsweise sichergestellt werden, dass der Rahmen durch die schweren Traggestelle zumindest nicht wesentlich verformt wird.

Alternativ oder zusätzlich zu mindestens einer Stütze kann die Traggestell-Haltevorrichtung auch ein oder mehrere andere Elemente aufweisen, welche ein Traggestell haltern und/oder stützen und/oder fixieren können. So kann die Traggestell-Haltevorrichtung auch beispielsweise ein oder mehrere Fixierelemente zum vollständigen oder teilweisen Fixieren mindestens eines Traggestells aufweisen. Beispielsweise kann die Traggestell-Haltevorrichtung mindestens eine Aufhängung aufweisen, an welcher oder mittels welcher mindestens ein Traggestell oder mindestens ein Teil desselben aufgehängt werden kann.

Wie oben ausgeführt, kann das Sortiment neben der genannten mindestens einen ersten Halterung und der mindestens einen zweiten Halterung weitere Halterungen umfassen. So kann das Sortiment beispielsweise weiterhin mindestens eine dritte Halterung zur Reinigung von Lungenautomaten aufweisen. Die dritte Halterung kann beispielsweise mindestens eine Lungenautomat-Haltevorrichtung zur Positionierung mindestens eines Lungenautomaten aufweisen. Die dritte Halterung kann insbesondere weiterhin mindestens eine Druckbeaufschlagungsvorrichtung mit mindestens einem Druckanschluss aufweisen, wobei der Druckanschluss mit dem Lungenautomaten verbindbar sein kann und wobei die Druckbeaufschlagungsvorrichtung eingerichtet sein kann, um den Lungenautomaten während einer Reinigung mit Druckgas zu beaufschlagen. Die dritte Halterung kann insbesondere derart ausgestaltet sein, dass diese keine Masken-Haltevorrichtung aufweist.

Weitere mögliche Ausgestaltungen betreffen die Ausgestaltung der dritten Halterung. So kann bezüglich der Lungenautomat-Haltevorrichtung hinsichtlich möglicher optionaler Ausgestaltungen weitgehend auf die obige Beschreibung der ersten Halterung und dort der Merkmale der Lungenautomat-Haltevorrichtung verwiesen werden. So kann die Lungenautomat-Haltevorrichtung der dritten Halterung beispielsweise wiederum mindestens eine Aufnahme aufweisen, in welcher ein Stutzen des Lungenautomaten einsteckbar ist und in welcher der Stutzen nach dem Einstecken fest gehaltert ist. Die Aufnahme kann insbesondere wiederum eine Reibbremse zur reibschlüssigen Halterung des Lungenautomaten umfassen.

Der Druckanschluss der dritten Halterung kann insbesondere wiederum mindestens eine Kupplung zum Ankoppeln eines Druckgasschlauchs des Lungenautomaten aufweisen. Die Kupplung kann insbesondere wiederum durch mindestens eine abnehmbare Kappe verschließbar sein, wenn kein Druckgasschlauch angeschlossen ist. Die Druckbeaufschlagungsvorrichtung der dritten Halterung kann insbesondere wiederum mindestens eine Druckluftzufuhr aufweisen, wobei die Druckluftzufuhr beispielsweise in eine Druckgasleitung der dritten Halterung münden kann, wobei der Druckanschluss auf die Druckgasleitung aufgesetzt sein kann. Die Druckluftzufuhr kann insbesondere mindestens einen Schlauch umfassen. Die Druckgasleitung kann insbesondere wiederum als tragendes Bauteil ausgestaltet sein und zumindest die Lungenautomat-Haltevorrichtung tragen. Die Druckgasleitung kann insbesondere wiederum als Rahmen ausgestaltet sein. Der Rahmen kann insbesondere wiederum ringförmig geschlossen sein.

Auch die dritte Halterung kann insbesondere eingerichtet sein, um eine Mehrzahl von Lungenautomaten aufzunehmen. Wie auch bei der ersten Halterung, ist bei der dritten Halterung besonders bevorzugt, wenn mindestens zwei, vorzugsweise mindestens vier und besonders bevorzugt mindestens acht Lungenautomaten aufgenommen werden können.

Die dritte Halterung kann ebenfalls wiederum mindestens einen Kleinteilkorb aufweisen. Bezüglich möglicher Ausgestaltungen des Kleinteilkorbs kann wiederum auf die obige Beschreibung der ersten Halterung verwiesen werden. Der Kleinteilkorb kann insbesondere wiederum zur Aufnahme von Zubehörteilen während eines Reinigungsvorgangs eingerichtet sein. Der Kleinteilkorb kann wiederum eine Mehrzahl von Öffnungen aufweisen, beispielsweise Öffnungen mit einem Äquivalentdurchmesser von 3 mm bis 20 mm, insbesondere von 5 mm bis 15 mm und besonders bevorzugt von 10 mm. Der Kleinteilkorb kann insbesondere wiederum ganz oder teilweise aus einem Drahtgeflecht hergestellt sein. Der Kleinteilkorb kann weiterhin wiederum insbesondere von der dritten Halterung abnehmbar sein. So kann die dritte Halterung beispielsweise mindestens eine Aufnahme zum Einsetzen des Kleinteilkorbs aufweisen, wobei die Aufnahme, wie auch im Fall der ersten Halterung, beispielsweise derart ausgestaltet sein kann, dass das Einsetzen des Kleinteilkorbs reversibel erfolgen kann.

Wiederum können auch im Fall der dritten Halterung eine Mehrzahl von Kleinteilkörben vorgesehen sein. Die Mehrzahl der Kleinteilkörbe kann beispielsweise wiederum durch einen gemeinsamen Deckel verschließbar sein.

Die dritte Halterung kann, wie oben ausgeführt, insbesondere zur Reinigung einer Mehrzahl von Lungenautomaten eingerichtet sein. Dabei kann beispielsweise jedem Lungenautomaten jeweils ein Kleinteilkorb zugeordnet sein. So kann beispielsweise wiederum die mindestens eine Aufnahme zum Einsetzen des mindestens einen Kleinteilkorbs derart ausgestaltet sein, dass jeweils ein Kleinteilkorb räumlich einer bestimmten Lungenautomat-Haltevorrichtung zuweist, so dass eine eindeutige Zuordnung gewährleistet werden kann. Auch auf diese Weise kann wiederum ein Untermischungsschutz gewährleistet werden.

Alternativ oder zusätzlich zu der dritten Halterung kann das Sortiment weiterhin mindestens eine vierte Halterung zur Reinigung von Atemmasken aufweisen. Diese vierte Halterung kann insbesondere wiederum mindestens eine Masken-Haltevorrichtung zur Positionierung mindestens einer Atemmaske aufweisen. Besonders bevorzugt ist es im Fall dieser vierten Halterung, wenn die vierte Halterung keine Lungenautomat-Haltevorrichtung aufweist. Bezüglich möglicher Ausgestaltungen der Masken-Haltevorrichtung kann wiederum weitgehend auf die obige Beschreibung der ersten Halterung verwiesen werden. Auch andere Ausgestaltungen sind jedoch grundsätzlich möglich.

So kann die Masken-Haltevorrichtung der vierten Halterung beispielsweise wiederum mindestens ein Gestell umfassen, wobei das Gestell beispielsweise wiederum mindestens einen Haltebügel für die mindestens eine Atemmaske aufweisen kann, wobei die Atemmaske auf den Haltebügel aufgestülpt werden kann. Der Haltebügel kann insbesondere wiederum derart eingerichtet sein, dass die Atemmaske derart auf den Haltebügel aufgestülpt werden kann, dass eine Innenseite der Atemmaske nach oben weist, beispielsweise schräg nach oben. Das Gestell kann wiederum beispielsweise ein Drahtgestell sein oder mindestens ein Drahtgestell umfassen. Der Haltebügel kann dementsprechend beispielsweise wiederum mindestens einen Drahtbügel umfassen.

Das Gestell der vierten Halterung kann beispielsweise wiederum mindestens einen weiteren Haltebügel für mindestens eine Innenmaske aufweisen.

Analog zur ersten Halterung kann auch die Masken-Haltevorrichtung der vierten Halterung optional ein oder mehrere Fixierelemente zum Fixieren der mindestens einen Atemmaske und/oder der mindestens einen optionalen Innenmaske aufweisen. Diese Fixierelemente können beispielsweise jeweils eine geöffnete Position und eine geschlossene Position aufweisen. In der geöffneten Position kann beispielsweise ein Einbringen der jeweiligen Atemmaske und/oder optionalen Innenmaske in die Masken-Haltevorrichtung und/oder eine Entfernung der jeweiligen Atemmaske und/oder optionalen Innenmaske aus der Masken-Haltevorrichtung erfolgen. In der geschlossenen Position kann beispielsweise eine Fixierung der jeweiligen Atemmaske und/oder optionalen Innenmaske erfolgen, beispielsweise um ein Verrutschen während eines Reinigungsvorgangs zu verhindern. Das mindestens eine optionale Fixierelement kann beispielsweise beweglich gelagert sein. Beispielsweise kann dieses einen Drahtbügel und/oder Drahtring umfassen. Beispielsweise kann jeweils ein Fixierelement für eine Innenmaske beweglich auf einen Drahtbügel und/oder eine andere Halterung einer zugehörigen Atemmaske aufgeschoben sein. Auch andere Ausgestaltungen sind jedoch denkbar, beispielsweise schwenkbare und/oder auf andere Weise verschiebbare Ausführungsformen.

Die vierte Halterung kann wiederum, wie auch die erste Halterung und die optionale dritte Halterung, mindestens einen Kleinteilkorb zur Aufnahme von Zubehörteilen während eines Reinigungsvorgangs aufweisen. Bezüglich möglicher Ausgestaltungen des Kleinteilkorbs kann beispielsweise wiederum auf die obige Beschreibung verwiesen werden. Auch andere Ausgestaltungen sind jedoch grundsätzlich möglich. So kann der Kleinteilkorb beispielsweise wiederum eine Mehrzahl von Öffnungen aufweisen, beispielsweise Öffnungen in einer, mehreren oder allen Wänden des Kleinteilkorbs, wobei durch diese Öffnungen Reinigungsfluid ins Innere des Kleinteilkorbs eindringen kann. Die Öffnungen können beispielsweise einen Äquivalentdurchmesser von 3 mm bis 20 mm, insbesondere von 5 mm bis 15 mm und besonders bevorzugt von 10 mm aufweisen. Auch andere Dimensionierungen sind jedoch grundsätzlich möglich. Wie auch im Fall des Kleinteilkorbs der ersten Halterung und/oder des Kleinteilkorbs der dritten Halterung, können die Öffnungen grundsätzlich einen beliebigen Querschnitt aufweisen, beispielsweise einen runden Querschnitt und/oder einen rechteckigen Querschnitt, beispielsweise einen quadratischen Querschnitt. Der Kleinteilkorb kann beispielsweise wiederum ganz oder teilweise aus einem Drahtgeflecht hergestellt sein, wobei jedoch wiederum auch, wie im obigen Falle, andere Materialien verwendbar sind.

Der Kleinteilkorb kann insbesondere wiederum von der ersten Halterung abnehmbar sein. So kann beispielsweise die vierte Halterung wiederum mindestens eine Aufnahme zum Einsetzen des Kleinteilkorbs aufweisen, beispielsweise eine Aufnahme in einem Gestell der vierten Halterung. Die vierte Halterung kann beispielsweise wiederum eine Mehrzahl von Kleinteilkörben aufweisen. Diese Mehrzahl von Kleinteilkörben können wiederum durch einen gemeinsamen Deckel verschließbar sein.

Die vierte Halterung kann insbesondere zur Reinigung einer Mehrzahl von Atemmasken eingerichtet sein, wobei jeder Atemmaske beispielsweise wiederum ein Kleinteilkorb zugeordnet sein kann. Beispielsweise kann dieses wiederum dadurch erfolgen, dass eine Mehrzahl von Masken-Haltevorrichtungen vorgesehen ist, wobei die Aufnahme derart eingerichtet ist, dass jeweils ein Kleinteilkorb einer Masken-Haltevorrichtung räumlich zuweist. Auf diese Weise kann wiederum ein Untermischungsschutz gewährleistet werden.

Ein weiterer möglicher Aspekt betrifft die Halterungen. So können die verschiedenen Halterungen, also die erste Halterung und die zweite Halterung sowie optional zusätzlich die dritte Halterung und/oder die vierte Halterung, derart ausgestaltet sein, dass diese hinsichtlich eines oder mehrerer Bauteile baugleich sind oder sogar bauteilidentisch, wobei in letzterem Fall beispielsweise ein Bauteil zwischen den Halterungen austauschbar ist.

Insbesondere können die Halterungen jeweils mindestens einen Grundkorb und mindestens einen Aufsatz aufweisen. So kann der Grundkorb der Halterungen, also der ersten Halterung und der zweiten Halterung sowie optional zusätzlich der dritten Halterung und/oder der vierten Halterung, baugleich sein oder sogar, wie unten noch näher ausgeführt wird, identisch und zwischen den Halterungen austauschbar sein. Dabei unterscheiden sich jedoch die Aufsätze der Halterungen voneinander, so dass beispielsweise die erste Halterung einen ersten Aufsatz aufweist, die zweite Halterung einen zweiten Aufsatz sowie optional die optionale dritte Halterung einen dritten Aufsatz und/oder optional die optionale vierte Halterung einen vierten Aufsatz. Dabei kann sich der erste Aufsatz von dem zweiten Aufsatz unterscheiden. Weiterhin können sich der erste Aufsatz und/oder der zweite Aufsatz jeweils von dem dritten Aufsatz und/oder dem vierten Aufsatz unterscheiden.

Unter einem Grundkorb ist dabei allgemein im Rahmen der vorliegenden Erfindung ein Korb zu verstehen, welcher die jeweilige Halterung nach unten abschließt und auf welchen der jeweilige Aufsatz ganz oder teilweise aufgesetzt werden kann und/oder in welchen der jeweilige Aufsatz ganz oder teilweise eingebracht werden kann. Wie unten noch näher ausgeführt wird, kann der Korb insbesondere ein Bodenteil mit einer Bodenfläche aufweisen sowie Korbwände, welche sich von dem Bodenteil nach oben hin erstrecken. Das Bodenteil und die Korbwände bilden gemeinsam eine Korbwand. Diese Korbwand kann, wie unten noch näher ausgeführt wird, insbesondere eine Mehrzahl von Öffnungen aufweisen.

Unter einem Aufsatz ist dementsprechend ein Funktionsbauteil zu verstehen, welches auf den Grundkorb aufgesetzt und/oder ganz oder teilweise in den Grundkorb eingebracht werden kann und welches reversibel und/oder dauerhaft mit dem Grundkorb verbunden sein oder verbunden werden kann. Dieses Funktionsteil kann beispielsweise jeweils ein oder mehrere Funktionselemente aufweisen, wie beispielsweise die oben beschriebene Masken-Haltevorrichtung und/oder die oben beschriebene Lungenautomat-Haltevorrichtung und/oder den oben genannten Druckanschluss und/oder die Traggestell-Haltevorrichtung und/oder andere der genannten Funktionselemente.

Wie oben ausgeführt, kann der Grundkorb beispielsweise mindestens ein Bodenteil aufweisen. Allgemein kann der Grundkorb, beispielsweise das Bodenteil, eine rechteckige Bodenfläche aufweisen. Der Grundkorb kann weiterhin einen von diesem Bodenteil nach oben weisenden Rand, beispielsweise einen rechtwinklig von dem Bodenteil nach oben stehenden Rand, aufweisen. Das Bodenteil kann beispielsweise eben ausgestaltet sein und kann derart ausgestaltet sein, dass dieses bei in einer Reinigungskammer aufgenommenem Grundkorb horizontal ausgerichtet ist. Der nach oben weisende Rand, welcher vorzugsweise ein umlaufender Rand ist, kann dementsprechend beispielsweise im Wesentlichen vertikal angeordnet sein, wenn der Grundkorb mit der jeweiligen Halterung in der Reinigungskammer aufgenommen ist.

Die Aufsätze können dann beispielsweise jeweils auf dem Rand des zugehörigen Grundkorbs aufgesetzt sein. Die Aufsätze können allgemein beispielsweise jeweils reversibel mit dem zugehörigen Grundkorb verbindbar sein. Beispielsweise können die Aufsätze reversibel mit dem Rand des Grundkorbs verbindbar sein. Alternativ ist jedoch grundsätzlich auch eine feste Verbindung möglich.

Zum Zweck einer Herstellung einer reversiblen Verbindung bietet sich insbesondere eine kraftschlüssige Verbindung an. So können die Aufsätze beispielsweise jeweils durch eine kraftschlüssige Verbindung mittels mindestens eines Verbindungselements, welches Bestandteil des jeweiligen Aufsatzes und/oder des Grundkorbs sein kann, mit dem zugehörigen Grundkorb verbunden sein oder verbindbar sein. Beispielsweise kann diese kraftschlüssige Verbindung eine Rastverbindung umfassen. Alternativ oder zusätzlich können jedoch grundsätzlich auch andere Verbindungsarten und/oder Kombinationen davon eingesetzt werden.

Wie oben ausgeführt, ist es besonders bevorzugt, wenn die Halterungen hinsichtlich mindestens eines Bauteils bauteilidentisch sind oder sogar mindestens ein Bauteil identisch in sämtlichen Halterungen verwendbar ist. Dies kann insbesondere der genannte Grundkorb sein. So kann insbesondere derselbe Grundkorb Bestandteil der Halterungen sein, wobei die Halterungen sich in ihren reversibel mit diesem Grundkorb verbindbaren Aufsätzen unterscheiden können. So können beispielsweise nacheinander in verschiedenen Reinigungsschritten unterschiedliche Aufsätze auf denselben Grundkorb aufgesetzt werden. Grundsätzlich ist jedoch auch eine Ausgestaltung möglich, bei welcher unterschiedliche Halterungen unterschiedliche Grundkörbe aufweisen, auch wenn diese Grundkörbe grundsätzlich baugleich sein können.

Wie oben ausgeführt, kann der Grundkorb insbesondere ein ebenes Bodenteil aufweisen. Weiterhin kann der Grundkorb einen Rand, vorzugsweise einen umlaufenden Rand, aufweisen.

Der Grundkorb kann insbesondere ganz oder teilweise hergestellt sein aus mindestens einem Material ausgewählt aus der Gruppe bestehend aus einem Metall, einem Drahtmaterial und einem Kunststoff. Auch andere Materialien sind grundsätzlich einsetzbar. Wird ein Metall verwendet, so kann dies insbesondere ein drahtförmiges Metall sein. Alternativ oder zusätzlich können jedoch auch Metallbleche eingesetzt werden.

Der Grundkorb kann, wie oben ausgeführt, insbesondere eine Vielzahl von Öffnungen aufweisen. Diese Öffnungen können eine Beaufschlagung des in der jeweiligen Halterung aufgenommenen Atemgeräts mit Reinigungsfluid von unten her ermöglichen und/oder können ein Ablaufen von Reinigungsfluid nach Beaufschlagung des Atemgeräts nach unten hin ermöglichen. Diese Öffnungen können grundsätzlich einen beliebigen Querschnitt haben, beispielsweise einen runden und/oder einen polygonalen, insbesondere einen rechteckigen, Querschnitt. Beispielsweise kann der Grundkorb ganz oder teilweise als Gitterkorb ausgestaltet sein. Die Öffnungen können allgemein vorzugsweise einen Äquivalentdurchmesser von 3 mm bis 20 mm aufweisen, insbesondere einen Äquivalentdurchmesser von 5 mm bis 15 mm und besonders bevorzugt einen Äquivalentdurchmesser von 10 mm.

Der Grundkorb kann insbesondere ein Maschengeflecht aufweisen. Insbesondere kann der Grundkorb zumindest teilweise aus einem Drahtgitter hergestellt sein. Wenn der Grundkorb ein Bodenteil sowie einen Rand aufweist, beispielsweise gemäß der oben beschriebenen Ausführungsform, so können sowohl das Bodenteil als auch der Rand des Grundkorbs zumindest teilweise aus dem Drahtgitter hergestellt sein. Auch andere Ausgestaltungen sind jedoch grundsätzlich möglich.

In einem weiteren Aspekt der vorliegenden Erfindung wird ein Reinigungssystem zur Reinigung von Atemgeräten vorgeschlagen, insbesondere von Lungenautomaten und/oder Atemmasken, beispielsweise Atemschutzmasken. Das Reinigungssystem umfasst mindestens eine Reinigungsvorrichtung, welche mindestens eine Reinigungskammer zur Aufnahme mindestens eines Atemgeräts aufweist, wobei die Reinigungsvorrichtung weiterhin mindestens eine Fluideinrichtung zur Beaufschlagung des Atemgeräts mit mindestens einem Reinigungsfluid aufweist. Weiterhin weist das Reinigungssystem mindestens ein erfindungsgemäßes Sortiment gemäß einer oder mehreren der oben beschriebenen Ausführungsformen und/oder gemäß einer oder mehreren der nachfolgend noch näher beschriebenen Ausführungsformen auf.

Das Reinigungssystem ist dabei derart ausgestaltet, dass die Halterungen des Sortiments wahlweise in der Reinigungskammer aufnehmbar sind. So kann die Reinigungskammer beispielsweise eine Halterung, beispielsweise mindestens eine Schiene und/oder eine Auflagefläche und/oder eine andere Art der Aufnahme aufweisen, in welche die Halterungen wahlweise einfügbar sind, vorzugsweise reversibel. Die Reinigungsvorrichtung ist weiterhin eingerichtet, um in mindestens einem Reinigungsschritt die erste Halterung zu verwenden und um in diesem Reinigungsschritt über den Druckanschluss mindestens einen Lungenautomaten, welcher in der ersten Halterung aufgenommen ist, mit Druckgas zu beaufschlagen.

Hinsichtlich möglicher Ausgestaltungen des Reinigungsgeräts, mit Ausnahme der Halterungen und/oder des Sortiments, kann beispielsweise auf die oben bereits genannte WO 2011/144518 verwiesen werden. Auch andere Ausgestaltungen sind jedoch grundsätzlich möglich.

So kann die Reinigungskammer beispielsweise grundsätzlich als geschlossene, geöffnete oder zu öffnende Reinigungskammer ausgestaltet sein. Besonders bevorzugt ist es, wenn die Reinigungskammer allseitig oder zumindest in zwei Dimensionen von einem Gehäuse umschlossen ist, welches vollständig geschlossen ausgestaltet sein kann, welches jedoch grundsätzlich auch eine oder mehrere Durchführungen aufweisen kann. Die Reinigungskammer kann insbesondere als starre Reinigungskammer ausgestaltet sein, also als Reinigungskammer, welche während eines Reinigungsvorgangs ihre Position und/oder Ausrichtung nicht verändert. Alternativ oder zusätzlich kann die Reinigungskammer jedoch grundsätzlich auch als bewegliche Reinigungskammer ausgestaltet sein, beispielsweise als schwenkbare und/oder drehbare Reinigungskammer, welche während eines Reinigungsvorgangs in der Reinigungsvorrichtung ihre Position und/oder Orientierung verändert, beispielsweise durch eine Drehung, eine Rotation, einen Schleudervorgang, einen Rüttelvorgang oder ähnliche Bewegungen. Insofern kann die Reinigungskammer beispielsweise als Spülkammer einer Spülmaschine ausgestaltet sein, und die Reinigungsvorrichtung kann als Spülmaschine ausgestaltet sein, und/oder die Reinigungskammer kann als Trommel einer Waschmaschine ausgestaltet sein, und die Reinigungsvorrichtung kann in Form einer Waschmaschine ausgestaltet sein. Beispielsweise können Geschirrspülmaschinen und/oder Wäschewaschmaschinen kommerzieller Art oder für den Haushaltsbereich erfindungsgemäß modifiziert werden.

Die Reinigungsvorrichtung weist weiterhin mindestens eine Fluideinrichtung zur Beaufschlagung des Atemgeräts mit mindestens einem Reinigungsfluid auf. Unter einem Reinigungsfluid ist dabei eine grundsätzlich beliebige Flüssigkeit und/oder ein grundsätzlich beliebiges Gas zu verstehen, welches einen makroskopisch und/oder mikroskopisch reinigenden Effekt auf das Atemgerät aufweisen kann. Unter einer Fluideinrichtung ist allgemein eine Vorrichtung zu verstehen, mittels derer das innerhalb der Reinigungskammer aufgenommene Atemgerät in beliebiger Weise direkt oder indirekt mit dem Reinigungsfluid beaufschlagt werden kann. Dies kann beispielsweise in Form eines direkten Beaufschlagens erfolgen, beispielsweise durch ein Besprühen, Betropfen, Bestrahlen oder eine Kombination der genannten und/oder anderer direkter Beaufschlagungsarten, bei welchen aus der Fluideinrichtung austretendes Reinigungsfluid unmittelbar auf das Atemgerät auftrifft. Diese Ausgestaltung der Beaufschlagung kann insbesondere bei fester Reinigungskammer erfolgen, beispielsweise bei einer Spülmaschine. Alternativ oder zusätzlich kann die Beaufschlagung mittels der Fluideinrichtung auch derart erfolgen, dass die Fluideinrichtung die Reinigungskammer vollständig oder teilweise mit Reinigungsfluid füllt, so dass das in der Reinigungskammer aufgenommene Reinigungsgut zumindest in einer Stellung der Reinigungskammer mit dem Reinigungsfluid in Kontakt kommt. Diese Ausgestaltung der Beaufschlagung kann insbesondere bei einer Ausgestaltung der Reinigungskammer als bewegliche Reinigungskammer genutzt werden, beispielsweise in Form einer Trommel. Auch Kombinationen der genannten Beaufschlagungsarten und/oder anderer Beaufschlagungsarten sind möglich. Unabhängig davon, ob die Reinigungskammer starr oder beweglich ausgestaltet ist, kann die Beaufschlagung mit dem Reinigungsfluid im einfachen Betrieb erfolgen, indem das Reinigungsfluid lediglich einmal das Atemgerät beaufschlagt. Alternativ oder zusätzlich kann jedoch auch eine Reinigung im Umwälzbetrieb erfolgen, indem Reinigungsfluid mehrfach auf das Atemgerät aufgebracht wird. Derartige Umwälzbetriebe und Umwälzkreisläufe sind beispielsweise aus herkömmlichen Spülmaschinen oder Waschmaschinen bekannt.

Die Reinigungsvorrichtung ist, wie oben ausgeführt, eingerichtet, um in mindestens einem Reinigungsschritt die erste Halterung zur verwenden und über den Druckanschluss mindestens einen in der ersten Halterung aufgenommenen Lungenautomaten mit Druckgas zu beaufschlagen. Zu diesem Zweck kann die Reinigungsvorrichtung mindestens eine vorrichtungsseitige Druckbeaufschlagungsvorrichtung aufweisen, die mit der Druckbeaufschlagungsvorrichtung der ersten Halterung zusammenwirkt, beispielsweise indem die vorrichtungsseitige Druckbeaufschlagungsvorrichtung mit der Druckbeaufschlagungsvorrichtung der ersten Halterung verbunden wird.

Unter einer Druckbeaufschlagungsvorrichtung ist allgemein im Rahmen der vorliegenden Erfindung eine Vorrichtung zu verstehen, welche eingerichtet ist, um ein Druckgas, also ein Gas, mit einem Druck oberhalb des Normaldrucks bereitstellen zu können, beispielsweise einem Druck von mindestens 1,5 bar, vorzugsweise einem Druck von mindestens 2 bar. Unter einem Druckanschluss ist grundsätzlich ein beliebiger Anschluss der Druckbeaufschlagungsvorrichtung zu verstehen, über welchen das Druckgas der Druckbeaufschlagungsvorrichtung bereitstellbar ist.

Die vorrichtungsseitige Druckbeaufschlagungsvorrichtung kann insbesondere im Inneren der Reinigungskammer angeordnet sein und/oder vom Inneren der Reinigungskammer aus zugänglich sein. Auch eine Bereitstellung einer Mehrzahl von Druckanschlüssen ist denkbar.

Die Druckbeaufschlagungsvorrichtung der ersten Halterung soll derart ausgestaltet sein, dass der mindestens eine Druckanschluss mit dem mindestens einen Lungenautomaten verbindbar ist. Zusätzlich zu dem Lungenautomaten können grundsätzlich auch ein oder mehrere andere gasführende Elemente mit dem Druckgas, beispielsweise Druckluft, Stickstoff und/oder ein anderes Druckgas, über die Druckbeaufschlagungsvorrichtung beaufschlagbar sein. Unter einem gasführenden Element ist dabei allgemein ein Element zu verstehen, welches bei einer Benutzung des Atemgeräts durch einen menschlichen oder tierischen Benutzer mit einem Atemgas beaufschlagt wird oder auf andere Weise mit dem Atemgas in Kontakt kommen kann. Insbesondere kann es sich hierbei um einen Lungenautomaten und/oder einen Atemschlauch mit mindestens einem Ventil handeln. Insbesondere kann es sich bei dem gasführenden Element um ein Element handeln, welches mindestens einen Schlauch und/oder mindestens eine andere Art von Gasführungsvorrichtung mit einem Innenraum und/oder mindestens ein Ventil aufweist, welche in der Regel nicht mit Reinigungsfluid beaufschlagt werden dürfen. Beispielsweise kann es sich bei dem gasführenden Element um einen Bereich des Atemgeräts handeln, welcher im Betrieb mit einem Druck oberhalb des Normaldrucks beaufschlagt wird, beispielsweise einen gasführenden Bereich eines Atemgeräts oberhalb eines Normaldrucks, beispielsweise oberhalb von 1,5 bar, insbesondere oberhalb von 2 bar.

Der Lungenautomat kann grundsätzlich beispielsweise ein einstufiger oder auch ein mehrstufiger Lungenautomat sein. Mit dem Druckgas können beispielsweise ein Teil einer ersten Stufe und/oder einer zweiten Stufe und/oder gegebenenfalls weiterer Stufen des Lungenautomaten beaufschlagt werden, oder auch ein vollständiger Lungenautomat. Beispielsweise kann zumindest ein Bereich zwischen einem Schlauchanschluss und einem Ventil des Lungenautomaten beaufschlagt werden.

Unter einer Verbindung zwischen dem Druckanschluss und dem Lungenautomaten ist allgemein eine Gasverbindung zu verstehen, so dass das Druckgas in den Lungenautomaten überführt werden kann, vorzugsweise ohne dass bei dieser Verbindung ein Verlust an Druckgas auftritt. Darüber hinaus kann optional die Verbindung zwischen dem Druckanschluss und dem Lungenautomaten mindestens eine mechanische Verbindung umfassen, insbesondere eine formschlüssige und/oder kraftschlüssige Verbindung, so dass der Lungenautomat fest mit dem Druckanschluss verbunden werden kann. Beispielsweise kann es sich hierbei um eine Schraubverbindung und/oder eine Klemmverbindung und/oder eine Spannverbindung handeln, wozu der Druckanschluss und/oder der Lungenautomat jeweils mindestens ein mechanisches Verbindungselement aufweisen können. Insbesondere kann es sich hierbei um eine Steckverbindung in Form einer Schnellkupplung und/oder um ein Gewinde handeln, beispielsweise ein Kupplungsstück für einen Atemanschluss des Lungenautomaten. Unter einer Schnellkupplung ist dabei allgemein eine gasdichte und/oder flüssigkeitsdichte Steckverbindung zwischen zwei Fluid-führenden Bauelementen zu verstehen, welche durch eine auf einfache und schnelle Weise herzustellende und zu lösende mechanische Fixierung, insbesondere ohne Verwendung eines Schraubverschlusses, mechanisch gesichert werden kann, beispielsweise durch mindestens einen Spannhaken und/oder einen Bajonettverschluss und/oder einen Schraubverschluss, beispielsweise eine Überwurfmutter. Die Verbindung kann insbesondere ohne Werkzeug herstellbar sein. Insbesondere kann der Druckanschluss eine Mehrzahl von fest oder austauschbar ausgestalteten Adaptern zur Verbindung mit unterschiedlichen Arten von Lungenautomaten umfassen. Mittels dieser Adapter können eine Mehrzahl unterschiedlicher Lungenautomaten direkt oder indirekt mit dem Druckanschluss verbindbar sein, beispielsweise unterschiedliche Arten und/oder unterschiedliche Typen von Lungenautomaten, beispielsweise unterschiedlicher Fabrikate oder Hersteller. Beispielsweise kann ein Adaptersatz mit einer Mehrzahl unterschiedlicher Kupplungsstücke (beispielsweise Kupplungsstücke einer Schnellkupplung) und/oder Gewinde zum Anschluss unterschiedlicher Lungenautomaten vorgesehen sein. Beispielsweise kann es sich hierbei um unterschiedliche Schnellkupplungssysteme und/oder Normgewinde handeln. Alternativ oder zusätzlich kann der mindestens eine Druckanschluss jedoch auch als fester Druckanschluss für einen bestimmten Typ von Verbindungselement bzw. für einen bestimmten Typ von gasführendem Element ausgestaltet sein. Besonders vorteilhaft ist es, wenn der Druckanschluss und/oder das Kupplungssystem so eingerichtet sind, dass einerseits kein Gas austreten kann, wenn kein Gegenstück angeschlossen ist, und dass in diesem Fall auch kein anderes Medium, beispielsweise Reinigungsfluid, in den Lungenautomaten eintreten kann.

Die optionale vorrichtungsseitige Druckbeaufschlagungsvorrichtung der Reinigungsvorrichtung ist eingerichtet, um, in Zusammenwirkung mit der Druckbeaufschlagungsvorrichtung der ersten Halterung sowie ggf. in einem weiteren Reinigungsschritt mit der Druckbeaufschlagungsvorrichtung der dritten Halterung, das gasführende Element mit Druckgas zu beaufschlagen. Unter einem Druckgas ist dabei allgemein ein beliebiges Gas zu verstehen, welches einen Druck oberhalb des Normaldrucks, also einen Druck oberhalb von 1 bar, aufweist. Insbesondere kann es sich dabei um einen Druck oberhalb von 1,5 bar, insbesondere oberhalb von 2 bar, und besonders bevorzugt oberhalb von 3 bar handeln. Besonders bevorzugt erfolgt die Druckbeaufschlagung mit dem Druckgas derart, dass sämtliches Reinigungsfluid aus einem mit dem Druckgas beaufschlagten Innenraum des Lungenautomaten ferngehalten wird. Bei dem Druckgas kann es sich beispielsweise um Druckluft oder ein anderes gasförmiges Medium mit einem Überdruck handeln, beispielsweise Stickstoff, Kohlendioxid oder ähnliches. Insbesondere kann ein Inertgas als Druckgas eingesetzt werden.

Die Reinigungsvorrichtung kann insbesondere derart ausgestaltet sein, dass die Druckbeaufschlagung über die Druckbeaufschlagungsvorrichtung während mindestens eines Reinigungsvorgangs erfolgt, beispielsweise während mindestens eines Reinigungsschritts oder Programmschritts eines einschrittigen oder mehrschrittigen Reinigungsprogramms. Insbesondere kann die Beaufschlagung mit dem Druckgas gleichzeitig zur Beaufschlagung des Atemgeräts mit dem Reinigungsfluid erfolgen.

Die Reinigungsvorrichtung kann insbesondere als Einkammerspülmaschine ausgestaltet sein und/oder eine Einkammerspülmaschine umfassen. Unter einer Einkammerspülmaschine ist dabei eine Spülmaschine mit einer einzelnen Reinigungskammer zu verstehen, in welcher vorzugsweise mehrere Reinigungsschritte (im Folgenden auch Programmschritte genannt) eines mehrstufigen Reinigungsprogramms ausgeführt werden können. Die Beaufschlagung innerhalb einer Einkammerspülmaschine kann insbesondere durch eine Fluideinrichtung in Form einer oder mehrerer Düsen erfolgen, beispielsweise in Form einer oder mehrerer Sprühdüsen und/oder anderer Düsen, beispielsweise starren und/oder drehbaren und/oder schwenkbaren Düsenarmen. Die Einkammerspülmaschine kann insbesondere als so genannte Mehrkreis-Spülmaschine ausgestaltet sein. Dementsprechend kann die Einkammerspülmaschine beispielsweise mindestens einen separat von der Reinigungskammer ausgebildeten Fluidtank aufweisen, wobei in dem Fluidtank mindestens ein Reinigungsfluid unabhängig von einem in der Reinigungsvorrichtung momentan ablaufenden Reinigungsprozess konditionierbar ist, beispielsweise erwärmbar und/oder mit Zusätzen beaufschlagbar ist. Derartige Spülmaschinen mit einem Mehrkreisprinzip sind aus dem Bereich gewerblicher Geschirrspülmaschinen bekannt, bei welchen, unabhängig von der Reinigungskammer, in der Regel ein Tank für eine Konditionierung einer Nachspülflüssigkeit vorgesehen ist, beispielsweise ein Boiler und/oder ein Tank mit einem Durchlauferhitzer.

Die Bereitstellung des Druckgases kann durch die Reinigungsvorrichtung selbst erfolgen und/oder durch eine externe Vorrichtung. So kann die vorrichtungsseitige Druckbeaufschlagungsvorrichtung der Reinigungsvorrichtung insbesondere mindestens einen externen Druckanschluss zur Verbindung mit einer externen Druckquelle aufweisen. Bei dieser externen Druckquelle kann es sich beispielsweise um eine getrennt von der Reinigungsvorrichtung ausgebildete Druckgasflasche und/oder eine bauseitige Druckgasleitung, beispielsweise eine Pressluftleitung, handeln. Alternativ oder zusätzlich kann die vorrichtungsseitige Druckbeaufschlagungsvorrichtung auch mindestens eine in die Reinigungsvorrichtung integrierte Druckgasquelle aufweisen. Insbesondere kann mindestens eine integrierte Druckgasflasche vorgesehen sein und/oder mindestens ein integrierter Kompressor, zur Bereitstellung des Druckgases. Alternativ oder zusätzlich zur Bereitstellung eines einzelnen Druckgases können auch mehrere Druckgase gleichzeitig oder nacheinander bereitgestellt werden.

Die Reinigungsvorrichtung kann insbesondere als Programmautomat eingerichtet sein, um ein Reinigungsprogramm mit einem oder mehreren Programmschritten oder Reinigungsschritten durchzuführen. Insbesondere kann die Reinigungsvorrichtung eingerichtet sein, um ein Reinigungsprogramm mit mindestens zwei unterschiedlichen Reinigungsschritten durchzuführen. Beispielsweise kann bei unterschiedlichen Reinigungsschritten eine Beaufschlagung mit unterschiedlichen Arten von Reinigungsfluiden erfolgen, beispielsweise mindestens ein Reinigungsschritt, in welchem eine Beaufschlagung mit einer Reinigerlösung erfolgt, und mindestens ein weiterer Reinigungsschritt, insbesondere ein Nachspülschritt, in welchem eine Beaufschlagung mit einem Nachspülfluid erfolgt. Optional kann gegebenenfalls auch mindestens ein Reinigungsschritt vorgesehen sein, welcher als Trocknungsschritt ausgestaltet ist, wobei die Trocknung gegebenenfalls passiv erfolgen kann, beispielsweise durch einfaches Abtropfen, oder, alternativ, bei welchem die Trocknung auch aktiv unterstützt werden kann, beispielsweise durch automatisiertes Ausblasen der Atemmasken mit Druckluft und/oder durch Vorsehen einer Wärmequelle wie Warmluft, Mikrowellen und/oder mindestens einer ähnlichen Wärme- oder Trocknungsquelle.

Weitere mögliche Ausgestaltungen betreffen die Fluideinrichtung. Wie oben ausgeführt, kann die Fluideinrichtung insbesondere mindestens eine Düse aufweisen. Insbesondere kann es sich dabei um eine Düse handeln, welche ausgewählt ist aus einer Sprühdüse, einer Spüldüse, einem Sprüharm, insbesondere einem schwenkbaren und/oder drehbaren Sprüharm, einer im Umwälzbetrieb betreibbaren Düse. Auch eine Kombination der genannten und/oder anderer Arten von Düsen ist denkbar. Alternativ oder zusätzlich kann die Fluideinrichtung jedoch auch beispielsweise eine einfache Öffnung zum Einlass von Reinigungsfluid in die Reinigungskammer umfassen.

Das Reinigungsfluid kann insbesondere ein wässriges Reinigungsfluid umfassen, also Wasser oder Wasser mit Zusatz von einem oder mehreren Zusätzen, welche in gelöster Form, in Emulsionsform oder auch in Suspensionsform vorliegen können. Alternativ oder zusätzlich kann das Reinigungsfluid auch mindestens eine Reinigerlösung umfassen. Unter einer Reinigerlösung ist dabei allgemein eine Lösung mindestens eines Tensids in mindestens einem Lösungsmittel, beispielsweise ebenfalls Wasser, zu verstehen. Beispielsweise kann es sich hierbei um eine kommerzielle Reinigerlösung handeln, welche auch für Geschirrspülmaschinen eingesetzt werden kann und/oder welche in Waschmaschinen eingesetzt werden kann. Auch spezielle Reinigerlösungen können jedoch grundsätzlich entwickelt und/oder eingesetzt werden. Wiederum alternativ oder zusätzlich kann das Reinigungsfluid auch ein Reinigungsfluid mit mindestens einem Klarspüler umfassen, also einem Zusatz, welcher ein Abtrocknen des Atemgeräts erleichtert und/oder ein Abperlen von Flüssigkeit von mindestens einer Oberfläche des Atemgeräts erleichtert. Derartige Klarspüler, welche ebenfalls ein oder mehrere Tenside umfassen können, sind aus dem Bereich der Geschirrspültechnik grundsätzlich bekannt. Wiederum alternativ oder zusätzlich kann das Reinigungsfluid auch mindestens ein Nachspülfluid umfassen, welches grundsätzlich beispielsweise Wasser sein kann und/oder Wasser mit einem oder mehreren Zusätzen. Wiederum alternativ oder zusätzlich kann das Reinigungsfluid auch ein Reinigungsfluid mit mindestens einem Desinfektionsmittel umfassen, wobei unter einem Desinfektionsmittel grundsätzlich eine beliebige Substanz verstanden werden kann, welche eine keimabtötende Wirkung aufweist. Derartige Desinfektionsmittel sind aus dem Stand der Technik ebenfalls grundsätzlich bekannt. Wiederum alternativ oder zusätzlich kann das Reinigungsfluid auch demineralisiertes Wasser umfassen. Wie unten noch näher ausgeführt wird, kann das demineralisierte Wasser beispielsweise in einer entsprechenden Demineralisierungsvorrichtung der Reinigungsvorrichtung bereitgestellt werden, beispielsweise einem Ionentauscher. Alternativ oder zusätzlich kann die Demineralisierungsvorrichtung jedoch auch beispielsweise eine Umkehrosmosevorrichtung umfassen. Wiederum alternativ oder zusätzlich kann das Reinigungsfluid auch ein erwärmtes Reinigungsfluid umfassen. Insbesondere zum Nachspülen können sich derartige erwärmte Reinigungsfluide eignen, wobei unter erwärmten Reinigungsfluiden grundsätzlich Reinigungsfluide zu verstehen sind, welche eine Temperatur von mindestens 25 °C aufweisen. Besonders bevorzugt sind Temperaturen von 30 °C bis 70 °C und insbesondere 60 °C. Diese Temperaturen haben sich insbesondere für die Reinigung von flexiblen Elastomerwerkstoffen, wie sie für Atemgeräte häufig eingesetzt werden, als geeignet erwiesen.

Wie oben dargestellt, ist für die Reinigung von Atemgeräten die Bereitstellung qualitativ hochwertiger Reinigungsfluide von besonderer Bedeutung. Auf diese Weise kann beispielsweise eine mikroskopische oder auch makroskopische Verunreinigung der Atemgeräte vermieden werden. Durch mikroskopische Verunreinigungen wie beispielsweise mineralische Verunreinigungen entstehen beispielsweise Kalkablagerungen auf Sichtflächen wie beispielsweise Sichtscheiben der Atemgeräte. Weiterhin können mikrobielle Verunreinigungen zu einer Kontamination der Atemgeräte führen. Besonders bevorzugt ist es daher, wenn die Reinigungsvorrichtung mindestens eine Umkehrosmosevorrichtung zur Bereitstellung von Wasser aufweist, insbesondere von demineralisiertem Wasser. Unter einer Umkehrosmosevorrichtung ist allgemein eine Vorrichtung zu verstehen, welche ein Fluid mit hohem Reinheitsgrad bereitstellen kann, unter Verwendung des Prinzips der Umkehrosmose. Beispielsweise kann die Umkehrosmosevorrichtung eine oder mehrere Osmosemembranen aufweisen, durch welche unter hohem Druck saubere Bestandteile des Wassers gepresst werden können, so dass ein aufgereinigtes Permeat entsteht, wohingegen auf einer Konzentratseite Verunreinigungen zurückbleiben. Beispielsweise können hierbei Drücke von oberhalb von 2 bar, insbesondere oberhalb von 5 bar, eingesetzt werden.

Wie oben dargestellt, können zum Zwecke der vorliegenden Erfindung insbesondere kommerziell erhältliche Wäschewaschmaschinen und/oder Geschirrspülmaschinen umgestaltet werden, beispielsweise gewerbliche Wäschewaschmaschinen und/oder gewerbliche Geschirrspülmaschinen. Diese Umgestaltung kann derart erfolgen, dass die Wäschewaschmaschinen bzw. Geschirrspülmaschinen mit einer vorrichtungsseitigen Druckbeaufschlagungsvorrichtung gemäß den oben beschriebenen Merkmalen ausgestattet werden. Die Reinigungsvorrichtung kann beispielsweise als Frontladerspülmaschine oder als Haubenspülmaschine ausgestaltet sein. Unter einer Frontladerspülmaschine ist dabei eine Spülmaschine mit einer Reinigungskammer zu verstehen, welche durch eine Klappe und/oder einen Schieber durch einen vor der Spülmaschine angeordneten Benutzer geöffnet werden kann. Unter einer Haubenspülmaschine ist dabei eine Spülmaschine zu verstehen, deren Reinigungskammer eine aufschwenkbare und/oder auffahrbare Haube umfasst, welche von sonstigen Bestandteilen der Reinigungskammer, beispielsweise einer Basis, getrennt werden kann. Derartige Haubenspülmaschinen sind grundsätzlich aus dem Bereich der gewerblichen Geschirrspültechnik bekannt. Die Reinigungskammer kann insbesondere starr ausgestaltet sein. Die Reinigungskammer kann allgemein während des Reinigungsvorgangs verriegelbar ausgestaltet sein, insbesondere indem eine automatische Verriegelung vorgesehen ist, welche vorzugsweise während eines Programms aktiviert wird und nach einem Programm automatisch wieder gelöst werden kann.

In einem weiteren Aspekt der vorliegenden Erfindung wird ein Verfahren zur Reinigung von Atemgeräten vorgeschlagen. Bei diesem Verfahren wird das Reinigungssystem gemäß der obigen Beschreibung oder gemäß den nachfolgend noch näher beschriebenen Ausführungsformen verwendet. Das Verfahren weist mindestens einen ersten Reinigungsschritt und mindestens einen zweiten Reinigungsschritt auf. In dem ersten Reinigungsschritt wird die erste Halterung verwendet, wobei mindestens eine Atemmaske in die Masken-Haltevorrichtung der ersten Halterung eingebracht wird. Dabei wird mindestens ein Lungenautomat in die Lungenautomat-Haltevorrichtung eingebracht. Über den Druckanschluss wird der Lungenautomat mit Druckgas beaufschlagt, und die Atemmaske und der Lungenautomat werden mittels der Fluideinrichtung mit dem Reinigungsfluid beaufschlagt.

In dem zweiten Reinigungsschritt wird die zweite Halterung verwendet. Dabei wird mindestens ein Traggestell für Druckgasflaschen für Atemgeräte in die Traggestell-Haltevorrichtung der zweiten Halterung eingebracht. Das Traggestell wird mittels der Fluideinrichtung mit dem Reinigungsfluid beaufschlagt.

Für weitere mögliche Einzelheiten des Verfahrens kann insbesondere auf die obige Beschreibung des Reinigungssystems verwiesen werden.

Das Sortiment, das Reinigungssystem und das Verfahren gemäß der vorliegenden Erfindung, in einer oder mehreren der oben beschriebenen Ausgestaltungen sowie gegebenenfalls in einer oder mehreren der nachfolgend noch näher beschriebenen Ausgestaltungen, weisen gegenüber bekannten Vorrichtungen und Verfahren der genannten Art zahlreiche Vorteile auf. So kann mittels des beschriebenen Sortiments ein Ladungsträger-System realisiert werden, welches Atemmasken und ihre verschiedenen Bestandteile derart aufnehmen kann, dass eine Reinigung und/oder eine Desinfektion optimal durchgeführt werden kann. Einzelteile der Atemgeräte, die zur Reinigung demontiert werden müssen, können derart in den Halterungen aufgenommen werden, dass sie den jeweiligen Atemmasken und/oder Lungenautomaten eindeutig zugeordnet werden können.

Allgemein kann mittels der vorliegenden Erfindung ein flexibles Korbsystem realisiert werden, welches speziell abgestimmt auf die Reinigung von Komponenten rund um die Atemgeräte ist. Das Korbsystem kann, wie oben ausgeführt, jeweils einen Grundkorb und spezielle Aufsätze umfassen, die mit dem Grundkorb lösbar verbunden sind. Optional können integrierte, zuordenbare Kleinteilbehälter in Form der Kleinteilkörbe, optional mit Deckel, beispielsweise in Form mindestens eines Abdeckgitters, vorgesehen sein.

Die Halterungen können vielseitig einsetzbar sein. So können diese beispielsweise zum Spülen von Teilen in einem Maskenspüler sowie auch zu einem anschließenden Trocknen in einen Trockenschrank eingesetzt werden. Der optionale Grundkorb kann beispielsweise ein flaches Bodenteil aufweisen, beispielsweise aus Drahtgitter. Auch eine seitliche Berandung kann aus einem Drahtgitter bestehen. Eine Öffnungsweite, beispielsweise eine Maschenweite, von 10 mm hat sich dabei in der Praxis als gut geeignet herausgestellt, so dass einerseits die zu reinigenden Güter optimal von Düsenstrahlen erreicht werden können und andererseits lose Bestandteile der Atemgeräte wie beispielsweise Haltebänder und/oder eine Bebänderung nicht mit den optionalen und in der Praxis vielfältig eingesetzten drehbaren Düsenarmen in Berührung kommen können und deren Bewegung behindern können.

Wie oben ausgeführt, können zu dem Grundkorb verschiedene Aufsätze verwendet werden, welche dann, gemeinsam mit dem Grundkorb, die jeweiligen Halterungen ergeben. So kann beispielsweise ein Aufsatz für vier Atemmasken und deren Einzelteile oder für mehr als vier Masken, beispielsweise acht Atemmasken, ohne Lungenautomaten, vorgesehen sein. Dieser Aufsatz, gemeinsam mit dem Grundkorb, kann beispielsweise die oben beschriebene vierte Halterung ergeben. Weiterhin kann ein Kombikorbaufsatz vorgesehen sein, beispielsweise für vier oder mehr Atemmasken sowie deren Einzelteile, mit Druckluftanschlüssen beispielsweise für vier oder mehr Lungenautomaten. Dieser Kombikorbaufsatz, gemeinsam mit dem Grundkorb, kann dann beispielsweise die oben beschriebene erste Halterung ergeben.

Weiterhin kann ein Korbaufsatz mit Druckluftanschlüssen für vier, sechs, acht oder mehr Lungenautomaten vorgesehen sein. Dieser Korbaufsatz kann dann beispielsweise, gemeinsam mit dem Grundkorb, die oben beschriebene dritte Halterung ergeben.

Weiterhin kann, wie oben ausgeführt, ein Korbaufsatz für ein Traggestell oder ein Korbaufsatz für mehrere Traggestelle vorgesehen sein. Dieser Korbaufsatz, gemeinsam mit dem beschriebenen Grundkorb, kann dann beispielsweise die oben genannte zweite Halterung ergeben.

Kleinteile der jeweiligen Atemmaske und/oder Kleinteile des jeweiligen Lungenautomaten können eindeutig einer Atemmaske und/oder einem Lungenautomaten zugeordnet werden. So kann beispielsweise mittels der Position eines Kleinteilkorbs und/oder einer Position der Halterung eine eindeutige Zuordnung erfolgen.

Die oben beschriebenen Aufsätze können vorteilhafterweise derart ausgebildet sein, dass diese auch auf marktübliche, gegebenenfalls bereits in handelsüblichen Reinigungsvorrichtungen und/oder Trockenschränken vorhandene Körbe aufgesetzt werden können. Die Grundkörbe und Korbaufsätze können grundsätzlich aus beliebigen Materialien gefertigt sein, beispielsweise aus Metall. Bauformen aus Kunststoff und/oder kombinierte Bauformen sind jedoch ebenfalls denkbar.

Durch die spezielle Ausbildung des Sortiments der Halterungen können alle Oberflächen der Atemgeräte sehr gut von dem Reinigungsfluid benetzt werden. Das Reinigungsfluid kann beispielsweise umgewälzt und/oder über Düsen versprüht werden. Durch eine optimale Halterung kann die mechanische Wirkung der Düsenstrahlen unterstützt werden.

Das Reinigungsfluid, beispielsweise die Spülflüssigkeit, kann von allen Seiten der zu reinigenden Teile ungehindert ablaufen, so dass die Verschmutzungen vollständig entfernt werden können. Durch das gute Ablaufen des Reinigungsfluids kann insbesondere ein nachfolgender Trocknungsprozess wesentlich erleichtert und damit auch verkürzt werden. Dies bedeutet in der Praxis neben einer Zeitersparnis eine bessere Ausnutzung der Trockenschränke und eine Einsparung von Heizenergie pro getrocknetem Atemgerät.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: eine Schnittdarstellung eines Ausführungsbeispiels einer Reinigungsvorrichtung zum Einsatz in einem erfindungsgemäßen Reinigungssystem;
- Figur 2: ein Ausführungsbeispiel eines Grundkorbs zum Einsatz in einem erfindungsgemäßen Sortiment;
- Figur 3: ein Ausführungsbeispiel eines ersten Aufsatzes zum Einsatz in einer ersten Halterung;
- Figur 4: ein Ausführungsbeispiel einer ersten Halterung;
- Figur 5: das Ausführungsbeispiel gemäß Figur 4 mit zusätzlich einer Mehrzahl von Kleinteilkörben;
- Figur 6: das Ausführungsbeispiel gemäß Figur 5 mit einer exemplarischen Bestückung mit einer Atemmaske, einer Innenmaske und einem Lungenautomaten;
- Figur 7: ein Ausführungsbeispiel eines zweiten Aufsatzes zur Verwendung in einer zweiten Halterung;
- Figur 8: ein Ausführungsbeispiel der zweiten Halterung;
- Figur 9: ein Bestückungsbeispiel der zweiten Halterung gemäß Figur 8 mit eingebrachtem Traggestell;
- Figur 10: ein Ausführungsbeispiel eines dritten Aufsatzes zur Verwendung in einer dritten Halterung;
- Figur 11: ein Ausführungsbeispiel einer dritten Halterung;
- Figur 12: ein Ausführungsbeispiel eines vierten Aufsatzes zur Verwendung in einer vierten Halterung; und
- Figur 13: ein Ausführungsbeispiel einer vierten Halterung.

### Ausführungsbeispiele

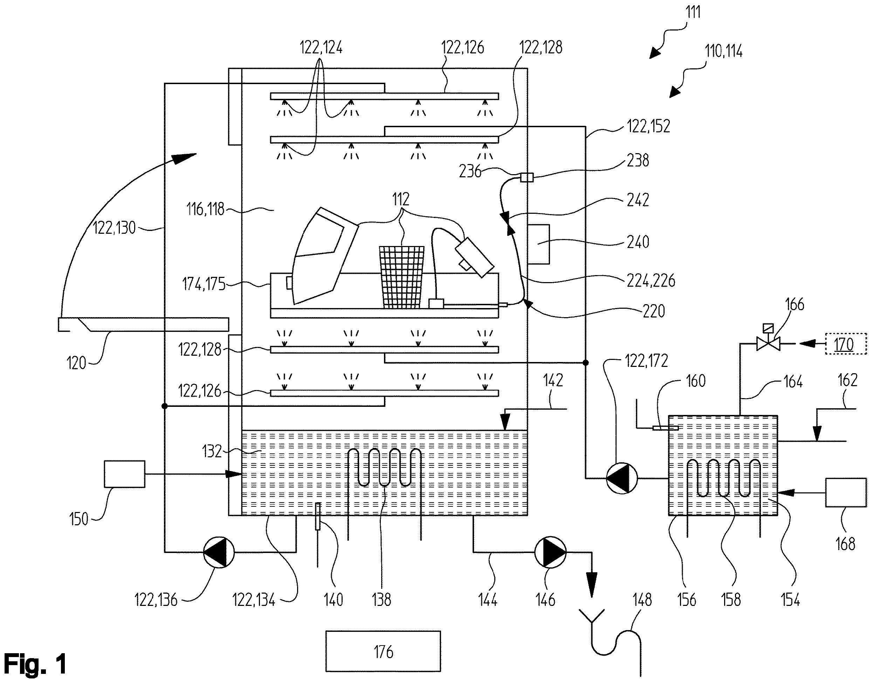

In Figur 1 ist ein Ausführungsbeispiel einer Reinigungsvorrichtung 110 dargestellt, welche zur Reinigung eines oder mehrerer Atemgeräte 112 eingerichtet ist und welche Bestandteil eines erfindungsgemäßen Reinigungssystems 111 ist. Die Reinigungsvorrichtung 110 ist in Figur 1 in einer Schnittdarstellung schematisch gezeigt. Die Reinigungsvorrichtung 110 kann beispielsweise als Spülmaschine 114 ausgestaltet sein. Die Reinigungsvorrichtung 110 umfasst eine Reinigungskammer 116, beispielsweise eine Spülkammer 118. Diese Reinigungskammer 116 kann beispielsweise durch eine Tür 120, beispielsweise eine Schwenktür, eine Schiebetür oder eine Klappe, und/oder durch eine andere Öffnungsvorrichtung geöffnet werden. Alternativ oder zusätzlich kann die Reinigungskammer 116 auch als durch eine Haube abgedeckte Reinigungskammer ausgestaltet sein. Auch andere Ausgestaltungen sind möglich. In dem in Figur 1 dargestellten Ausführungsbeispiel ist die Spülmaschine 114 beispielsweise als FrontladerSpülmaschine ausgestaltet. Auch andere Ausgestaltungen sind jedoch möglich.

Die Reinigungsvorrichtung 110 weist mindestens eine Fluideinrichtung 122 zur Beaufschlagung der in der Reinigungskammer 116 aufgenommenen Atemgeräte 112 mit einem oder mehreren Reinigungsfluiden auf. Beispielsweise kann die Fluideinrichtung 122 eine oder mehrere Düsen 124 umfassen, welche beispielsweise oberhalb und/oder unterhalb der Atemgeräte 112 und/oder an anderen Orten innerhalb der Reinigungskammer 116 angeordnet sein können, beispielsweise an einer oder mehreren Seitenwänden. Beispielsweise kann die Fluideinrichtung 122, wie in dem dargestellten Ausführungsbeispiel gemäß Figur 1, ein Waschdüsensystem 126 umfassen, beispielsweise mit einem oder mehreren unterhalb und/oder oberhalb der Atemgeräte 112 aufgenommenen, vorzugsweise drehbar und/oder schwenkbar gelagerten Düsenarmen mit mehreren Düsen 124. Alternativ oder zusätzlich kann die Fluideinrichtung 122 auch ein Nachspüldüsensystem 128 umfassen, beispielsweise mit einem oder mehreren Nachspüldüsenarmen, welche vorzugsweise wiederum drehbar und/oder schwenkbar gelagert sind, welche beispielsweise wiederum oberhalb und/oder unterhalb der Atemgeräte 112 angeordnet sein können. Auch andere Anordnungen und/oder Ausgestaltungen sind jedoch grundsätzlich möglich.

Die Fluideinrichtung 122 kann darüber hinaus ein oder mehrere weitere Elemente umfassen, wie beispielsweise eine oder mehrere Rohrleitungen, eine oder mehrere Pumpen und/oder ein oder mehrere Tanks. So ist beispielsweise in dem dargestellten Ausführungsbeispiel mindestens ein Waschleitungssystem 130 vorgesehen, zur Beaufschlagung des Waschdüsensystems 126 mit Reinigungsfluid 132, beispielsweise Reinigerlösung, aus einem oder mehreren Waschtanks 134. Beispielsweise kann ein Waschtank 134 im Bodenbereich der Reinigungskammer 116 vorgesehen sein und/oder auf andere Weise mit der Reinigungskammer 116 verbunden sein, so dass Reinigungsfluid 132 nach Beaufschlagung der Atemgeräte 112 wieder zurück in den Waschtank 134 fließen und/oder tropfen kann. Zur Beaufschlagung des Waschdüsensystems 126 mit dem Reinigungsfluid 132 aus dem Waschtank 134 kann die Fluideinrichtung 122 weiterhin eine oder mehrere Umwälzpumpen 136 aufweisen. Weiterhin können ein oder mehrere Heizelemente 138 vorgesehen sein, um das Reinigungsfluid 132 des Waschtanks 134 und/oder anderer Tanks zu erwärmen, beispielsweise in Form einer Waschtankheizung innerhalb des Waschtanks 134. Zur Kontrolle der Aufheizung des Reinigungsfluids 132 können ein oder mehrere Temperatursensoren 140 vorgesehen sein, beispielsweise innerhalb des Waschtanks 134. Weiterhin können ein oder mehrere Niveausensoren 142 vorgesehen sein, beispielsweise ein Niveausensor 142 als Niveausensor des Waschtanks 134. Der Waschtank 134 kann beispielsweise über eine Ablaufleitung 144 und optional über eine Ablaufpumpe 146 in einen Ablauf 148 entleerbar sein. Optional können auch ein oder mehrere, in Figur 1 nicht dargestellte, Zuläufe zu dem Waschtank 134 vorgesehen sein, um diesen mit Reinigungsfluid 132 zu befüllen. Alternativ oder zusätzlich kann eine Befüllung jedoch auch über das nachfolgend noch näher beschriebene Nachspüldüsensystem 128 erfolgen. Weiterhin kann ein Dosiersystem 150 vorgesehen sein, beispielsweise mindestens ein Dosiersystem, um ein oder mehrere Zusätze in das Reinigungsfluid 132 des Waschtanks 134 einzubringen, beispielsweise ein Reinigerkonzentrat, ein Klarspülmittel, ein Desinfektionsmittel oder Kombinationen der genannten und/oder anderer Zusätze.

Das Reinigungsfluid 132 kann insbesondere in einem Umwälzbetrieb auf die Atemgeräte 112 aufgebracht werden, indem das Reinigungsfluid 132 aus dem Waschtank 134 über das Waschdüsensystem 126 auf die Atemgeräte 112 aufgesprüht und/oder aufgespritzt wird, um dann wieder in den Waschtank 134 abzulaufen oder abzutropfen, um von dort aus erneut verwendet zu werden. Optional können ein oder mehrere Filter, beispielsweise Grobfilter und/oder Feinfilter, vorgesehen sein, um das Reinigungsfluid 132 des Waschtanks 134 zumindest teilweise aufzureinigen.

Das Nachspüldüsensystem 128 kann beispielsweise über mindestens ein Nachspülleitungssystem 152 mit einem weiteren Reinigungsfluid 154, beispielsweise einer Nachspülflüssigkeit, beaufschlagt werden. Dabei ist in Figur 1 eine optionale Ausgestaltung gezeigt, bei welcher die Reinigungsvorrichtung 110 als Zweikreissystem ausgestaltet ist. Dementsprechend wird das zweite Reinigungsfluid 154 aus einem separaten Tank bereitgestellt, welcher in dem dargestellten Ausführungsbeispiel als Nachspültank 156 ausgestaltet ist, der getrennt von dem Waschtank 134 ausgebildet ist. Beispielsweise kann dieser Nachspültank 156 als Boiler ausgestaltet sein und kann beispielsweise eine Nachspültankheizung 158 umfassen. Alternativ oder zusätzlich zu einer Nachspültankheizung 158 können auch andere Arten von Heizelementen für das zweite Reinigungsfluid 154 vorgesehen sein, beispielsweise ein oder mehrere Durchlauferhitzer. Selbiges gilt auch für das erste Reinigungsfluid 132 in dem Waschtank 134. Wiederum können in dem Nachspültank 156 ein oder mehrere Temperatursensoren 160 und/oder ein oder mehrere Niveausensoren 162 vorgesehen sein, und der Nachspültank 156 kann über einen oder mehrere Zuläufe 164 mit Reinigungsfluid 154 beschickt werden, beispielsweise Frischwasser. Der mindestens eine Zulauf 164 kann ein oder mehrere Ventile 166 aufweisen. Beispielsweise kann der Zulauf 164 mit einem bauseitigen Frischwasseranschluss verbunden oder verbindbar sein. Alternativ oder zusätzlich kann, bauseitig oder als Bestandteil der Reinigungsvorrichtung 110, mindestens eine Umkehrosmosevorrichtung 170 vorgesehen sein, über welche der Nachspültank 156 und/oder ein oder mehrere andere Tanks der Reinigungsvorrichtung 110 mit einem Permeat, beispielsweise aufgereinigtem Wasser, beaufschlagt werden können. Weiterhin kann wiederum mindestens ein Dosiersystem 168 vorgesehen sein, über welches ein oder mehrere Zusätze zu dem Reinigungsfluid 154 in dem Nachspültank 156 beigemischt werden können, beispielsweise ein oder mehrere Klarspülerkonzentrate.

Die Beaufschlagung des Nachspüldüsensystems 128 kann vorzugsweise im einfachen Betrieb erfolgen, also nicht im Umwälzbetrieb, so dass das Nachspülfluid aus dem Nachspültank 154 das Atemgerät 112 lediglich einmal beaufschlagt. Zur Beaufschlagung kann die Fluideinrichtung 122 beispielsweise ein oder mehrere Drucksteigerungspumpen 172 umfassen.

Die Reinigung der Atemgeräte 112 in der Reinigungsvorrichtung 110 gemäß Figur 1 kann beispielsweise dadurch erfolgen, dass zunächst ein oder mehrere Atemgeräte 112 mittels einer geeigneten Halterung 174, welche unten noch näher erläutert wird und welche ebenfalls Bestandteil des Reinigungssystems 111 ist, in die Reinigungskammer 116 eingebracht werden. Das Reinigungssystem 111 umfasst neben der Reinigungsvorrichtung 110 weiterhin ein Sortiment 175 verschiedener Halterungen 174, welche nachfolgend noch näher erläutert werden und welche wahlweise in die Reinigungskammer 116 eingebracht werden können. Es sind jedoch auch Reinigungsvorrichtungen 110 denkbar, bei welchen mehrere Halterungen 174 gleichzeitig in die Reinigungskammer 116 eingebracht werden können.

Nach dem Einbringen der Halterung 174 in die Reinigungskammer 116 kann die Tür 120 verschlossen werden, und es kann vorzugsweise ein Reinigungsprogramm gestartet werden, welches beispielsweise über eine Steuerung 176, beispielsweise eine zentrale Maschinensteuerung oder eine dezentrale Steuerung, gesteuert werden kann. Dabei kann beispielsweise zunächst der Waschtank 136 mittels des Nachspüldüsensystems 128 mit Reinigungsfluid 132 und/oder einer Vorstufe dieses Reinigungsfluids 132 befüllt werden, beispielsweise Frischwasser, insbesondere demineralisiertem Frischwasser. Dieses kann dann innerhalb des Waschtanks 134 konditioniert werden, beispielsweise durch Beimengung von einem oder mehreren Zusätzen über das Dosiersystem 150 und/oder durch Erwärmung mittels des Heizelements 138. Alternativ oder zusätzlich kann das Reinigungsfluid 132 auch nach einem Klarspülprogramm eines vorangehenden Reinigungszyklus im Waschtank 134 verblieben sein, um in einem sich anschließenden Reinigungszyklus als Reinigungsfluid 132 und/oder als Bestandteil desselben genutzt zu werden, da Nachspülfluid in der Regel auch nach Beaufschlagung des Atemgeräts 112 einen vergleichsweise hohen Reinheitsgrad aufweist.

Anschließend kann das Atemgerät 112, vorzugsweise im Umwälzbetrieb, in einem oder mehreren Waschprogrammschritten gereinigt, insbesondere gewaschen, werden. Hierbei können anhaftende Verunreinigungen von den Atemgeräten 112 entfernt werden, und/oder es kann eine Hygienisierung der Atemgeräte 112 erfolgen.

Anschließend an den mindestens einen Waschprogrammschritt können vorzugsweise ein oder mehrere Nachspülschritte durchgeführt werden. Hierzu kann der Waschtank 134 über die Ablaufleitung 144 und die Ablaufpumpe 146 optional entleert werden. Bereits während des mindestens einen Waschprogrammschritts kann der Nachspültank 156 mit dem Nachspülfluid 154, beispielsweise Frischwasser mit oder ohne Zusätze, befüllt worden sein, beispielsweise demineralisiertem Frischwasser. Anschließend können eine oder mehrere Zusätze über das Dosiersystem 168 beigemischt werden und/oder es kann eine Erwärmung des Reinigungsfluids 154 als Nachspülfluid mittels der Nachspültankheizung 158 und/oder eines Durchlauferhitzers erfolgen. Das derart vorkonditionierte Nachspülfluid 154 kann dann in dem mindestens einen Nachspülschritt über das Nachspüldüsensystem 128 auf die Atemgeräte 112 aufgebracht werden, so dass diese nachgespült und/oder klargespült werden. Nach dem mindestens einen Nachspülschritt kann sich optional wiederum mindestens ein Trocknungsschritt anschließen, welcher passiv ausgestaltet werden kann, durch einfaches Abwarten, oder welcher auch aktiv unterstützt werden kann, beispielsweise über mindestens ein Trocknungsgebläse und/oder eine andere Art von Trocknungsvorrichtung der Reinigungsvorrichtung 110, beispielsweise ein Infrarotstrahlersystem und/oder ein Mikrowellenstrahlungssystem. Verschiedene Ausgestaltungen sind denkbar. Anschließend an den optionalen Trocknungsschritt kann die bis dahin optional vorzugsweise verriegelte Tür 120 automatisch freigegeben und/oder geöffnet werden. Der gesamte Programmablauf kann beispielsweise durch die Steuerung 176 gesteuert werden, wobei auch mehrere Programmabläufe wählbar sein können.

Es wird darauf hingewiesen, dass das in Figur 1 dargestellte Ausführungsbeispiel der Reinigungsvorrichtung 110 lediglich eines von mehreren verschiedenen Ausführungsbeispielen darstellt. So können einzelne oder mehrere oder auch alle der oben beschriebenen Elemente auch in anderem Rahmen umgesetzt sein. Die Spülkammer 118 kann, alternativ oder zusätzlich zu der starren, ortsfesten Ausgestaltung gemäß Figur 1, auch schwenkbar und/oder drehbar ausgestaltet werden. Weiterhin kann auch der dargestellte Fluidkreislauf erheblich modifiziert werden.

Wie oben dargestellt, wird das mindestens eine Atemgerät 112 innerhalb der Reinigungsvorrichtung 110 vorzugsweise mittels mindestens einer Halterung 174 gehaltert. Dabei sind im Rahmen des Reinigungssystems 111 verschiedene derartiger Halterungen 174 vorgesehen. In Figur 1 ist lediglich schematisch ein mögliches Ausführungsbeispiel einer derartigen Halterung 174 dargestellt.

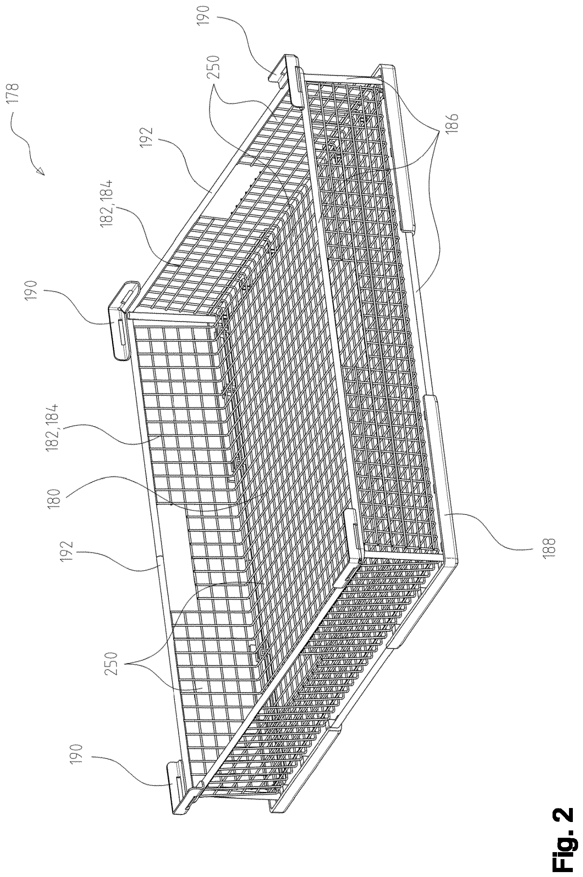

Ausführungsbeispiele möglicher Halterungen 174 eines möglichen Sortiments 175 an Halterungen 174 sind in den Figuren 2 bis 13 dargestellt. Den nachfolgenden Ausführungsbeispielen ist gemeinsam, dass diese einen in Figur 2 exemplarisch gezeigten Grundkorb 178 sowie verschiedene Aufsätze aufweisen. Der Grundkorb 178 kann beispielsweise reversibel mit den Aufsätzen verbunden sein und kann in sämtlichen Halterungen 174 eingesetzt werden.

Der in Figur 2 exemplarisch dargestellte Grundkorb 178 weist ein Bodenteil 180 und Seitenwände 182 in Form eines hochgezogenen Randes 184 auf. Das Bodenteil 180 kann beispielsweise als ebenes Bodenteil ausgestaltet sein, beispielsweise mit einer rechteckigen Grundfläche, und kann beispielsweise einen Rahmen 186 mit einem Kantenschutz 188 aufweisen. Am oberen, umlaufenden Rand der Seitenwände 182 können Positionierungshilfen 190 vorgesehen sein, in welche die nachfolgend näher beschriebenen Aufsätze eingesetzt werden können. Der Grundkorb 178 kann beispielsweise Traggriffe 192 aufweisen. Der Grundkorb kann beispielsweise aus einem Drahtgitter hergestellt sein, beispielsweise mit einer lichten Maschenweite von 10 mm. Auch andere Ausgestaltungen sind jedoch grundsätzlich möglich. Diesbezüglich kann beispielsweise auf die obige Beschreibung verwiesen werden.

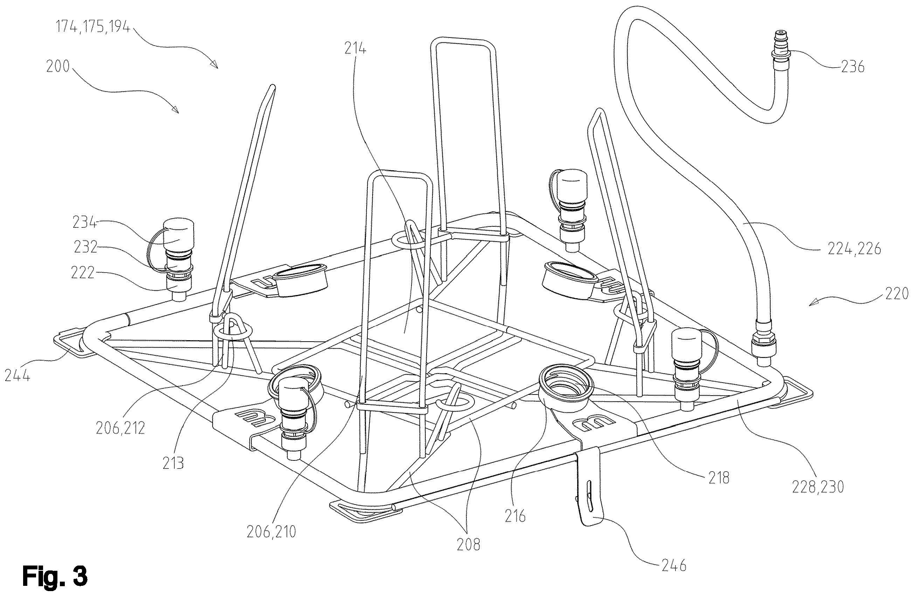

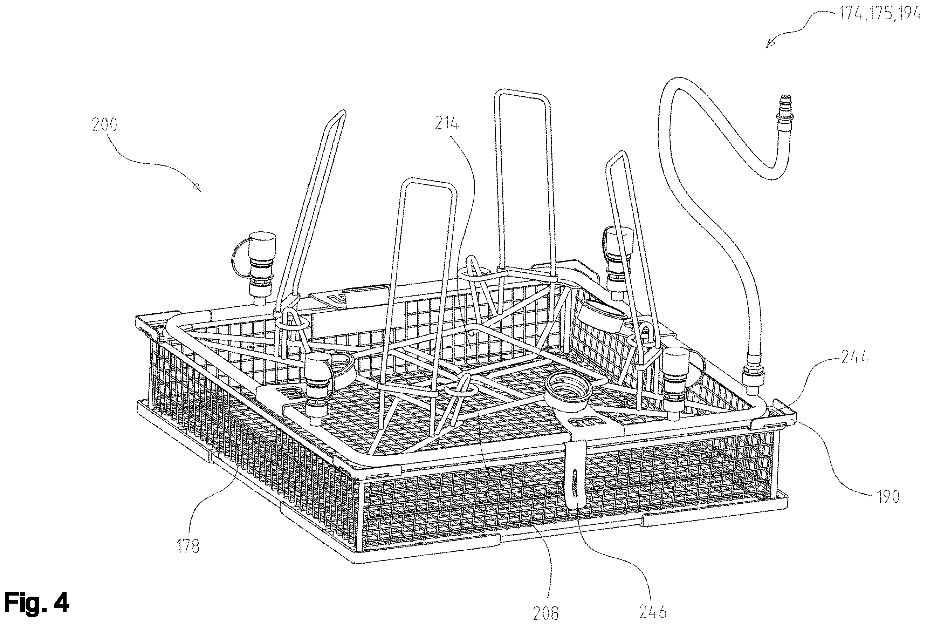

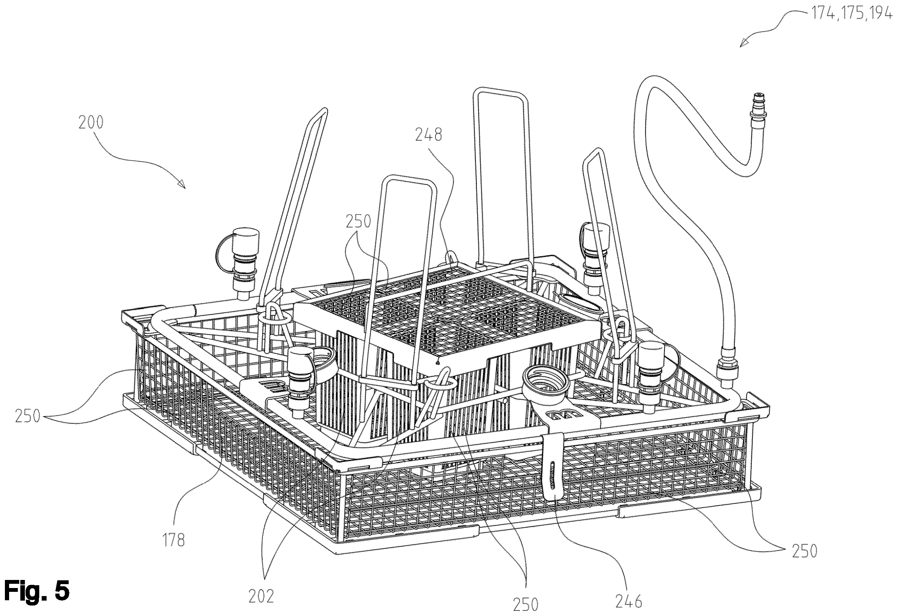

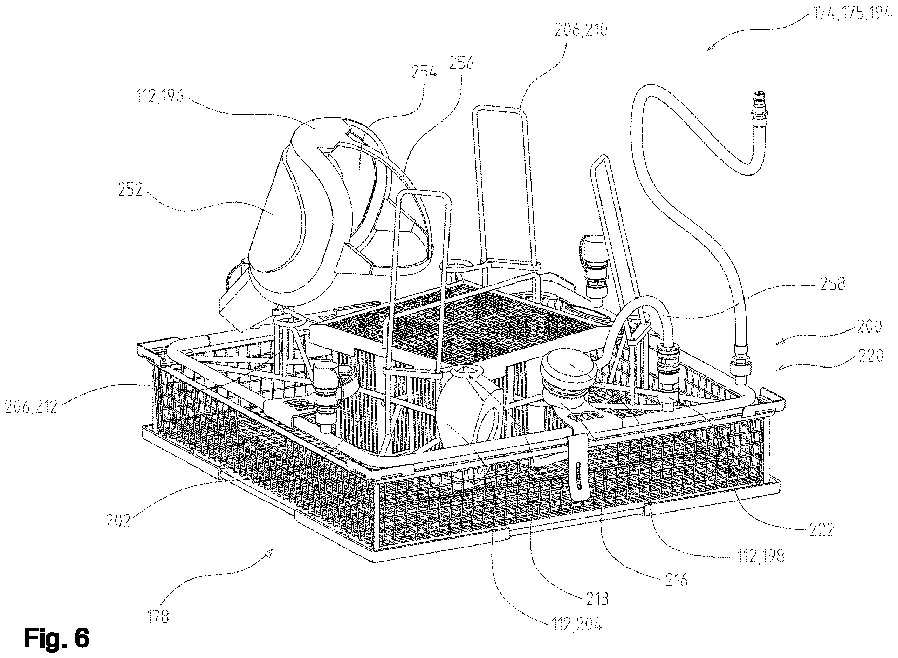

Wie oben ausgeführt, weist das Sortiment 175 mindestens zwei Halterungen 174 auf. In den Figuren 3 bis 6 ist, in verschiedenen Darstellungen, ein Ausführungsbeispiel einer möglichen ersten Halterung 194 des Sortiments 175 dargestellt. Diese Figuren werden im Folgenden im Wesentlichen gemeinsam erläutert. Die erste Halterung 194 dient zur gleichzeitigen Reinigung von Atemmasken 196 und Lungenautomaten 198. Dabei zeigt Figur 3 ein Ausführungsbeispiel eines ersten Aufsatzes 200, welcher auf den Grundkorb 178 gemäß Figur 2 aufgesetzt werden kann, Figur 4 zeigt die erste Halterung 194 nach Aufsetzen des ersten Aufsatzes 200 auf den Grundkorb 178, Figur 5 zeigt eine Ergänzung des Ausführungsbeispiels gemäß Figur 4 durch eine Mehrzahl von Kleinteilkörben 202, und Figur 6 zeigt das Ausführungsbeispiel gemäß Figur 5, wobei exemplarisch eine Atemmaske 196, eine Innenmaske 204 und ein Lungenautomat 198 in der ersten Halterung 194 aufgenommen sind.

Die erste Halterung 194 und dort insbesondere der erste Aufsatz 200 weist, wie in Figur 3 dargestellt, Plätze für jeweils vier Atemmasken 196, vier Innenmasken 204 und vier Lungenautomaten 198 auf. Auch eine andere Anzahl von Plätzen kann jedoch vorgesehen sein.

So weist der erste Aufsatz 200 Masken-Haltevorrichtungen 206 auf. Diese sind in Form eines Gestells 208 realisiert, welches Haltebügel 210 für die Atemmasken 196 sowie Haltebügel 212 für Innenmasken 204 aufweist.

Optional können die Masken-Haltevorrichtungen 206 in diesem oder auch in anderen Ausführungsbeispielen der Erfindung auch auf andere Weise gestaltet werden oder können, beispielsweise zusätzlich zu den Haltebügeln 212 für die Innenmasken 204, auch ein oder mehrere weitere Elemente aufweisen. So kann beispielsweise, wie in Figur 3 dargestellt, jedem Haltebügel 212 oder einer Gruppe mehrerer Haltebügel 212 jeweils mindestens ein Niederhalter 213 zugeordnet sein.

Dieser Niederhalter 213 kann beispielsweise ein bewegliches Element sein oder umfassen, welches beispielsweise eine geöffnete Position und eine geschlossene Position aufweisen kann. In der geöffneten Position kann beispielsweise mindestens eine Innenmaske 204 auf mindestens einen Haltebügel 212 aufgebracht werden, und/oder es kann mindestens eine Innenmaske 204 von dem mindestens einen Haltebügel 212 entnommen werden. In der geschlossenen Position kann beispielsweise gewährleistet sein, dass die Innenmaske 204 während eines Reinigungsvorgangs fixiert wird, so dass diese beispielsweise nicht von ihrem jeweiligen Haltebügel 212 abrutschen kann. In Figur 3 ist eine geschlossene Position des Niederhalters 213 gezeigt. Um den Niederhalter 213 in eine geöffnete Position zu bringen, kann dieser beispielsweise nach oben verschoben werden. Das Überführen des mindestens einen optionalen Fixierelements, beispielsweise des Niederhalters 213, von der geschlossenen in die geöffnete Position oder umgekehrt kann beispielsweise manuell und/oder auch automatisch erfolgen, Letzteres beispielsweise durch mindestens einen optionalen, entsprechend eingerichteten Aktor der Reinigungsvorrichtung 110.

Der Niederhalter 213 kann beispielsweise ganz oder teilweise mit einem oder mehreren der Haltebügel 210 und/oder 212 verbunden sein, vorzugsweise beweglich. So kann beispielsweise jeweils ein Niederhalter 213 verschiebbar auf jeweils einem Haltebügel 212 oder Haltebügel 210 (letztere Option ist in Figur 3 dargestellt) aufgebracht sein. Auf diese Weise kann der Niederhalter 213 beispielsweise durch einfaches Verschieben von einer geschlossenen Position in eine geöffnete Position oder umgekehrt überführt werden.

Der Niederhalter 213 ist lediglich ein Beispiel für mögliche Fixierelemente zum Fixieren der mindestens einen Atemmasken 196 und/oder der mindestens einen optionalen Innenmaske 204. Alternativ oder zusätzlich zu einem Niederhalter 213 können in diesem oder auch in anderen Ausführungsbeispielen auch ein oder mehrere andere Arten von Fixierelementen zum Fixieren der Atemmasken 196 und/oder der Innenmasken 204 vorgesehen sein.

Weiterhin kann das Gestell 208 eine Mehrzahl von Aufnahmen 214 für die Kleinteilkörbe 202 aufweisen.

Weiterhin weist der erste Aufsatz 200 in diesem Ausführungsbeispiel eine Mehrzahl von Lungenautomat-Haltevorrichtungen 216 auf. In diesem Ausführungsbeispiel sind exemplarisch vier derartiger Lungenautomat-Haltevorrichtungen 216 vorgesehen. Diese können beispielsweise jeweils eine runde Aufnahme aufweisen, in welche ein Stutzen des Lungenautomats einsteckbar ist. Innerhalb dieser Aufnahmen der Lungenautomat-Haltevorrichtung 216 können jeweils Reibbremsen 218 vorgesehen sein, beispielsweise zur reibschlüssigen Halterung der Lungenautomaten 198. Diese Reibbremsen 218 lassen sich beispielsweise durch Dichtringe aus einem Elastomermaterial realisieren.

Weiterhin weisen die erste Halterung 194 und der erste Aufsatz 200 eine Druckbeaufschlagungsvorrichtung 220 mit in diesem Fall einer Mehrzahl von Druckanschlüssen 222 auf. In dem dargestellten Ausführungsbeispiel weist die Druckbeaufschlagungsvorrichtung 220 eine Druckluftzufuhr 224 in Form eines Schlauchs 226 auf, welcher in einer Druckgasleitung 228 mündet. Diese Druckgasleitung 228 ist in diesem Ausführungsbeispiel als tragendes Bauteil ausgestaltet und bildet einen Rahmen 230. Dieser Rahmen 230, welcher ringförmig ausgestaltet ist und einen rechteckigen Querschnitt aufweist, sitzt in diesem Ausführungsbeispiel auf dem Gestell 208 auf, und die Lungenautomat-Haltevorrichtungen 216 sind auf diesen Rahmen 230 aufgeschoben und vorzugsweise angeschweißt. Alternativ oder zusätzlich zu einem Anschweißen können auch andere Verbindungsarten vorgesehen sein, beispielsweise formschlüssige und/oder kraftschlüssige Verbindungen und/oder eine Verklebung. Weiterhin sind auf diesen Rahmen 230 die Druckanschlüsse 222 aufgesetzt, so dass vorzugsweise eine durchgehende Fluidverbindung von der Kupplung 236 über den Schlauch 226 und die Druckgasleitung 228 bis zur Kupplung 232 am Druckanschluss 222 gebildet wird.

Die Druckanschlüsse 222 umfassen an ihrem oberen Ende jeweils eine Kupplung 232, an welche jeweils ein Druckgasschlauch eines Lungenautomaten 198 anschließbar ist. Diese Kupplung 232 ist, wenn kein Lungenautomat angeschlossen ist, durch eine abnehmbare Kappe 234 verschließbar.

Der Schlauch 226 weist an seinem freien Ende ebenfalls eine Kupplung 236 auf. Mit dieser Kupplung 236 ist, wie in Figur 1 schematisch dargestellt, ein Anschluss des Schlauchs 226 an eine vorrichtungsseitige Druckbeaufschlagungsvorrichtung 238 der Reinigungsvorrichtung 110 möglich. Hierbei kann es sich beispielsweise um einen Druckgasanschluss, beispielsweise einen Druckluftanschluss, handeln, welcher mit einer gebäudeseitigen Druckversorgung verbindbar oder verbunden ist, und/oder die Reinigungsvorrichtung 110 kann eine integrierte Druckgasquelle 240, beispielsweise eine Druckluftflasche und/oder einen Kompressor, umfassen. Weiterhin können in dem Schlauch 226 und/oder zwischen den Druckbeaufschlagungsvorrichtungen 220, 238 ein oder mehrere Ventile 242 vorgesehen sein.

Der in Figur 3 dargestellte erste Aufsatz 200 weist an seinen Ecken jeweils erweiterte Auflageecken 244 auf. Mittels dieser Auflageecken, welche beispielsweise Bestandteil des Gestells 208 sein können und/oder mit diesem Gestell 208 verbunden sein können, und an welchen beispielsweise auch der Rahmen 230 auf dem Gestell 208 aufliegen kann, wird der erste Aufsatz 200 auf den Grundkorb 178 und dort insbesondere auf die Positionierungshilfen 190 aufgesetzt. Dies ist beispielsweise in dem zusammengesetzten Zustand gemäß Figur 4 erkennbar. Vorzugsweise wird dabei der erste Aufsatz 200 lediglich auf den Grundkorb 178 aufgesetzt. Zusätzlich können dabei ein oder mehrere Verbindungselemente 246 vorgesehen sein, welche die Verbindung zwischen dem Grundkorb 178 und dem ersten Aufsatz 200 festigen. In dem dargestellten Ausführungsbeispiel ist beispielsweise an dem Gestell 208 ein Verbindungselement 246 in Form eines Rasthakens vorgesehen, so dass beispielsweise eine Rastverbindung zwischen dem Grundkorb 178 und dem ersten Aufsatz 200 herstellbar ist. Auch andere Ausgestaltungen sind jedoch möglich.

Der erste Aufsatz 200 oder, wahlweise, andere Aufsätze, können beispielsweise derart mit dem Grundkorb 178 verbunden werden, dass der jeweilige Aufsatz zunächst auf den Grundkorb 178 aufgesetzt wird. Die Auflageecken 244 können beispielsweise jeweils einen oder mehrere Fortsätze und/oder Zungen aufweisen, welche in passende Schlitze und/oder Öffnungen an den Positionierungshilfen 190 eingreifen können. An dieser Seite der jeweiligen Halterung kann sich dann beispielsweise eine formschlüssige Verbindung zwischen dem jeweiligen Aufsatz und den Grundkorb 178 ergeben. Das Verbindungselement 246 kann beispielsweise als Rasthaken ausgestaltet sein oder mindestens einen Rasthaken aufweisen, welcher zum Herstellen einer Verbindung beispielsweise oben in den Rand 184 am Grundkorb 178 eingreifen kann. Auf diese Weise oder auf andere Weise kann beispielsweise mindestens eine Rastverbindung hergestellt werden. Zum Lösen der Verbindung, insbesondere zum Trennen des jeweiligen Aufsatzes vom Grundkorb 178, kann beispielsweise das Verbindungselement 246 bewegt und/oder verformt werden. Beispielsweise kann dieses Verbindungselement 246 manuell nach außen geklappt und/oder geschwenkt werden, um die Verbindung zu lösen. Alternativ oder zusätzlich sind jedoch auch andere Ausgestaltungen denkbar.

In dem in Figur 4 dargestellten Zustand ist die erste Halterung 194 grundsätzlich gebrauchsfertig. Allerdings bietet es sich in vielen Fällen an, wie oben bereits erwähnt, zusätzlich Kleinteilkörbe 202 vorzusehen, welche in die Aufnahmen 214 in dem Gestell 208 eingesetzt werden können. In Figur 5 ist dementsprechend die erste Halterung 194 gemäß Figur 4 mit zusätzlich in die Aufnahmen 214 eingebrachten Kleinteilkörben 202 dargestellt. Diese Kleinteilkörbe 202 können beispielsweise durch einen gemeinsamen Deckel 248 verschließbar sein, um ein Herausfallen der Kleinteile zu verhindern. Die Aufnahmen 214 sind dabei derart in dem Gestell 208 positioniert, dass diese jeweils einer Masken-Haltevorrichtung 206 und einer Lungenautomat-Haltevorrichtung 216 zuweisen. In diesem Fall ist jedem Paar, bestehend aus einer Atemmaske 196 und einem Lungenautomaten 198, jeweils ein Kleinteilkorb 202 zugeordnet, so dass ein Untermischungsschutz gewährleistet ist.

Sowohl der Grundkorb 178 als auch die Kleinteilkörbe 202 sowie optional der Deckel 248 weisen jeweils eine Vielzahl von Öffnungen 250 auf, wie exemplarisch in Figur 5 dargestellt ist. Diese Öffnungen 250 können beispielsweise Maschenöffnungen in einem Drahtgeflecht des Grundkorbs, vorzugsweise sowohl im Bodenteil 180 als auch in den Seitenwänden 182, sein. Bei den Kleinteilkörben 202 können die Öffnungen 250 beispielsweise schlitzförmige Öffnungen in den Seitenwänden und/oder Öffnungen in einem (nicht näher dargestellten) Bodenteil der Kleinteilkörbe 202 sein. Im Deckelteil 248 können die Öffnungen 250 beispielsweise in Form von Maschenöffnungen ausgestaltet sein. Bezüglich des Grundkorbs 178 sind beispielsweise Maschenweiten von ca. 10 mm bevorzugt. Die Öffnungen 250 ermöglichen einerseits ein gleichmäßiges Eindringen von Sprühstrahlen des Reinigungsfluids 132 und andererseits ein Ablaufen desselben nach Beaufschlagung des Atemgeräts 112.

In Figur 6 ist schließlich die erste Halterung 194 in einem Zustand gezeigt, in welchem exemplarisch eine Atemmaske 196 auf einen Haltebügel 210 einer Masken-Haltevorrichtung 206 aufgestülpt ist, derart, dass eine Sichtscheibe 252 derselben schräg nach außen weist und dass eine Innenseite 254 nach oben weist. Haltebänder 256 der Atemmaske 196 können dabei lose ins Innere der ersten Halterung 194 ragen.

Weiterhin ist, wie oben ausgeführt, eine Innenmaske 204 exemplarisch auf einen Haltebügel 212 aufgesetzt. Weiterhin kann optional wiederum mindestens ein Niederhalter 213 und/oder eine andere Art von Fixierelement für die mindestens eine Innenmaske 204 und/oder auch für die Atemmaske 196 vorgesehen sein. Dies ist wiederum exemplarisch in Figur 6 dargestellt. Beispielsweise kann wiederum, wie oben beschrieben, in einem geschlossenen Zustand (wie dargestellt) jeweils mindestens ein Niederhalter 213 für jeweils eine Innenmaske 204 von oben an der Innenmaske 204 anliegen, um während eines Reinigungsvorgangs ein Verrutschen der Innenmaske 204 zu verhindern. Zum Platzieren und/oder herausnehmen der Innenmaske 204 kann der Niederhalter 213 beispielsweise in eine geöffnete Position gebracht werden, beispielsweise indem dieser nach oben verschoben wird. Zudem ist ein Lungenautomat 198 in eine Lungenautomat-Haltevorrichtung 216 eingesteckt, und ein Luftschlauch 258, auch als Druckgasschlauch des Lungenautomaten 198 bezeichnet, ist exemplarisch an einen Druckanschluss 222 angekoppelt.

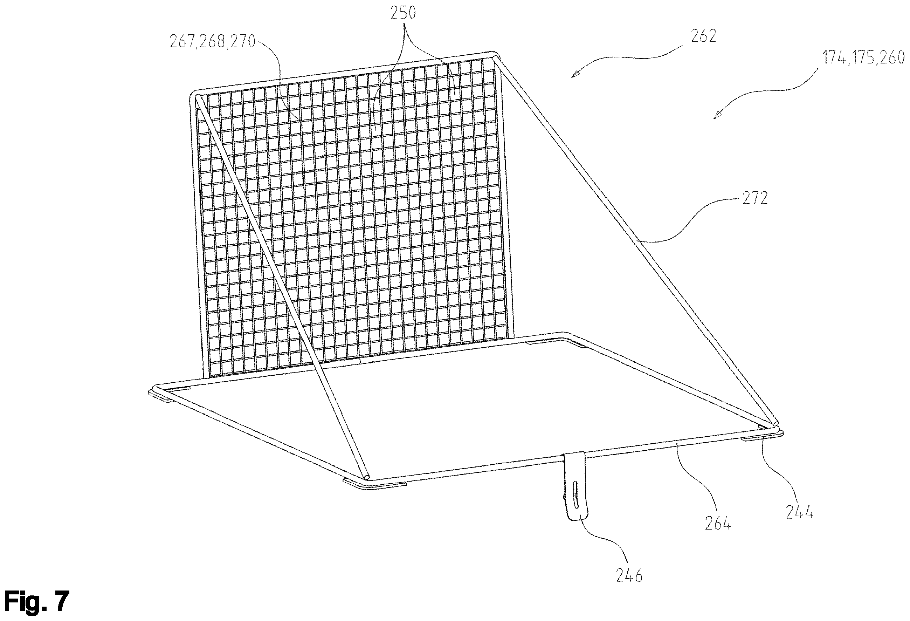

In den Figuren 7 bis 9 ist exemplarisch ein Ausführungsbeispiel einer zweiten Halterung 260 des Sortiments 175 gezeigt. Dabei zeigt Figur 7 wiederum einen zweiten Aufsatz 262, welcher in einen Grundkorb 178 gemäß Figur 2 eingesetzt werden kann. Zu diesem Zweck weist der zweite Aufsatz 262 einen Rahmen 264 auf, welcher rechteckig ist und im Wesentlichen der Grundfläche des Grundkorbs 178 entspricht. An den Ecken dieses Rahmens 264 können wiederum Auflageecken 244 vorgesehen sein, welche in die Positionierungshilfen 190 des Grundkorbs 178 einsetzbar sind. Weiterhin kann wiederum mindestens ein Verbindungselement 246 zur Verbindung mit dem Grundkorb vorgesehen sein. Bezüglich möglicher Ausgestaltungen dieses Verbindungselements 246 kann beispielsweise auf die obige Beschreibung der Figur 4 verwiesen werden.

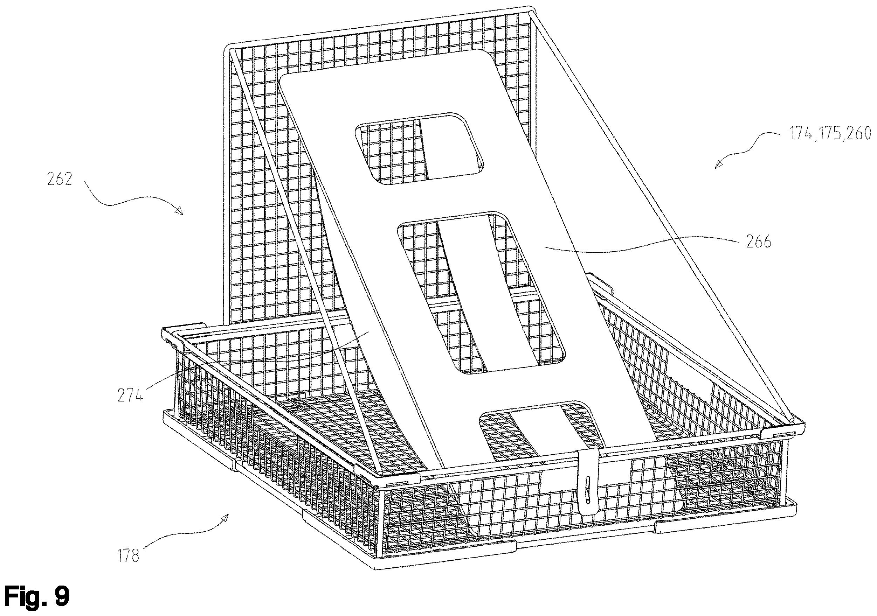

Die zweite Halterung 260 dient zur Halterung von Traggestellen 266 für Druckgasflaschen. Diese Traggestelle 266 sind exemplarisch in Figur 9 gezeigt. Um diese Traggestelle 266 zu stützen, weist der zweite Aufsatz 262 eine senkrechte Stütze 268 in Form beispielsweise einer ebenen Platte 270 auf, welche mit dem Rahmen 264 verbunden ist und welche über Querstreben 272 mit dem Rahmen 264 verbunden und stabilisiert ist. Die Stütze 268 bildet in diesem Fall ein Ausführungsbeispiel einer Traggestell-Haltevorrichtung 267. Die ebene Platte 270 ist beispielsweise als Drahtgeflecht ausgestaltet und weist eine Mehrzahl von Öffnungen 250 auf, beispielsweise wiederum Öffnungen mit einer Maschenweite von 10 mm. Auf diese Weise ist wiederum eine optimale Beaufschlagung der Traggestelle 266 mit Reinigungsfluid 132 von allen Seiten möglich.

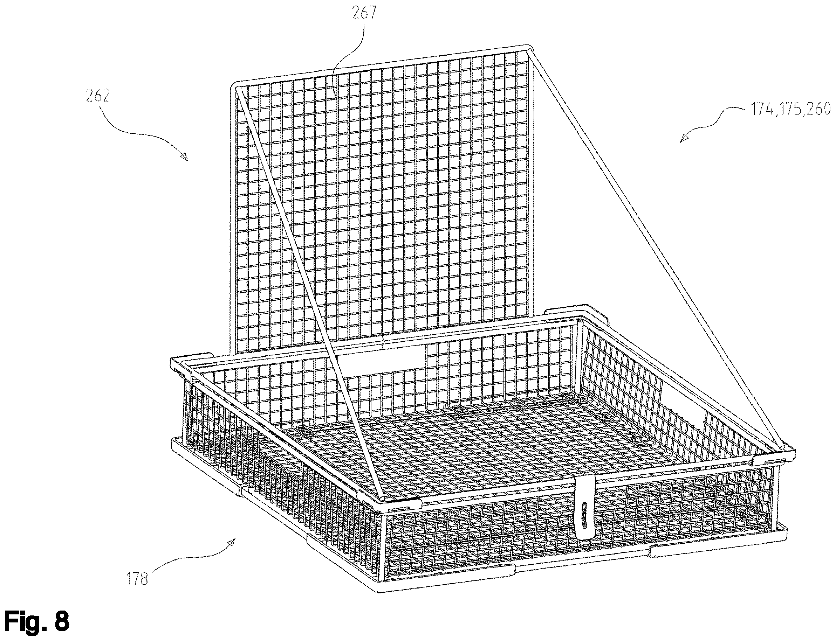

In Figur 8 ist die zweite Halterung 260 in einem zusammengesetzten Zustand gezeigt, in welchem der zweite Aufsatz 262 auf den Grundkorb 178 aufgesetzt ist. In Figur 9 ist schließlich, wie oben erwähnt, ein Zustand gezeigt, bei welchem ein Traggestell 266 in die zweite Halterung 260 eingebracht ist. Das Einbringen kann dabei derart erfolgen, dass das Traggestell 266 schräg zur Horizontalen in der zweiten Halterung aufgenommen ist, wobei Tragriemen 274 lose nach unten, zum Grundkorb 178 hin aufgenommen sein können.

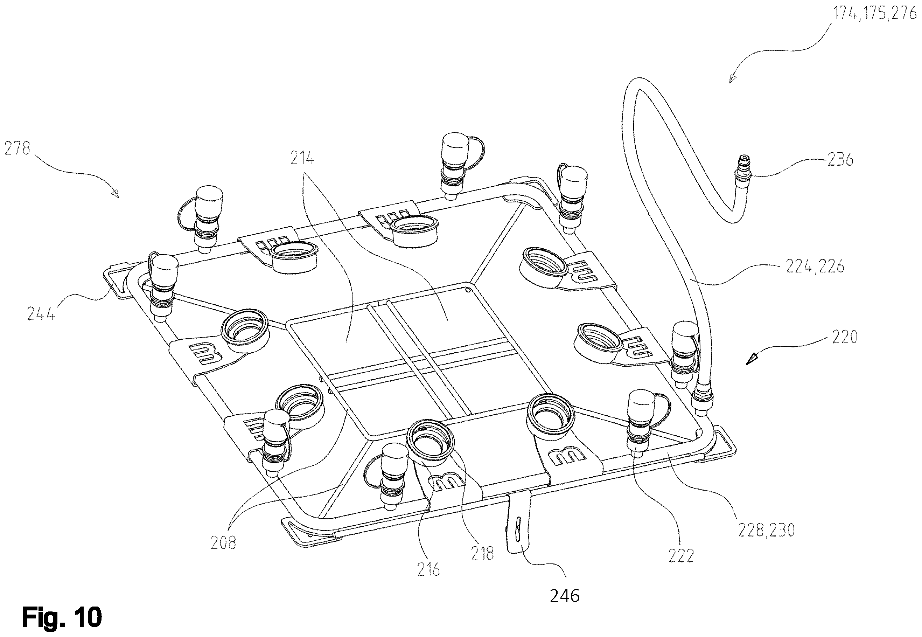

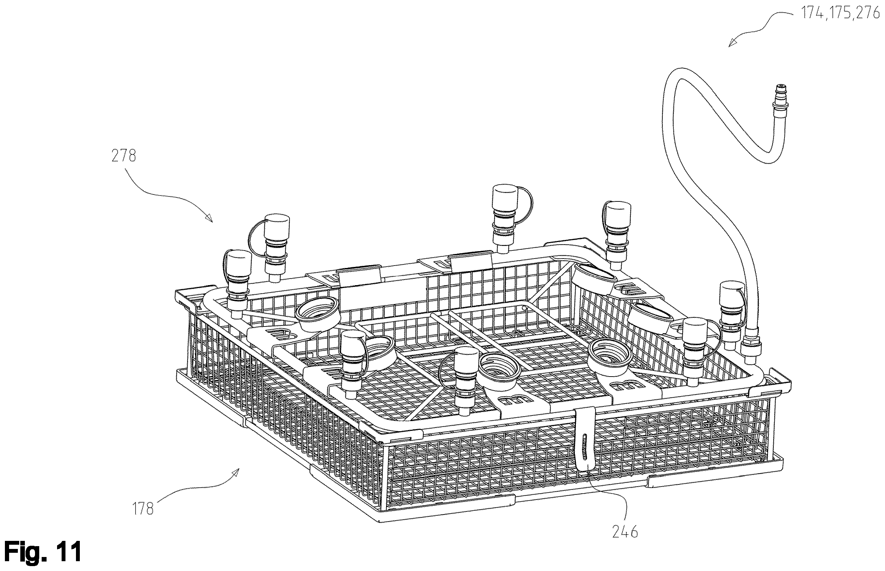

In den Figuren 10 und 11 ist ein Ausführungsbeispiel einer optionalen dritten Halterung 276 des Sortiments 175 gezeigt. Dabei zeigt Figur 10 einen dritten Aufsatz 278, welcher auf einen Grundkorb 178, beispielsweise gemäß Figur 2, aufgesetzt werden kann. Figur 11 zeigt einen zusammengesetzten Zustand.

Der dritte Aufsatz 278 entspricht zunächst im Wesentlichen dem ersten Aufsatz 200, beispielsweise gemäß Figur 3. Allerdings weist dieser dritte Aufsatz 178 keine Masken-Haltevorrichtung 206 auf. Dementsprechend dienen die dritte Halterung 276 und der dritte Aufsatz 278 zum Reinigen von Lungenautomaten 198, nicht hingegen zum Reinigen von Atemmasken 196. Für die Beschreibung des dritten Aufsatzes 278 kann weitgehend auf die obige Beschreibung verwiesen werden. So weist der dritte Aufsatz 278 wiederum eine Mehrzahl von Lungenautomat-Haltevorrichtungen 216 auf, wobei für jede Lungenautomat-Haltevorrichtung 216 ein Druckanschluss 222 einer Druckbeaufschlagungsvorrichtung 220 vorgesehen ist, an welchen, analog beispielsweise zur Darstellung gemäß Figur 6, ein Luftschlauch 258 eines Lungenautomaten 198 anschließbar ist. Die Druckbeaufschlagungsvorrichtung 220 weist wiederum, analog zu Figur 3, eine Druckluftzufuhr 224 in Form eines Schlauchs 226 auf, mit einer Kupplung 236 zum Anschluss an eine vorrichtungsseitige Druckbeaufschlagungsvorrichtung 238 gemäß Figur 1, sowie eine Druckgasleitung 228 in Form eines Rahmens 230, auf welchen die Druckanschlüsse 222 aufgesetzt sind. Der Rahmen 230 ist wiederum ringförmig geschlossen ausgestaltet und weist eine rechteckige Gestalt auf. In diesem Ausführungsbeispiel sind exemplarisch an jeder Seite des Rahmens 230 zwei Lungenautomat-Haltevorrichtungen 216 und zwei Druckanschlüsse 222 vorgesehen, so dass insgesamt acht Lungenautomaten 198 gleichzeitig gereinigt werden können. Auch andere Ausgestaltungen sind jedoch möglich. Der Rahmen 230 kann wiederum als tragendes Bauteil ausgestaltet sein und kann die Lungenautomat-Haltevorrichtungen 216 tragen. Der Rahmen 230 kann beispielsweise wiederum auf einem Gestell 208 aufsitzen, welches an seinen Ecken Auflageecken 244 aufweist, die in die Positionierungshilfen 190 des Grundkorbs 178 eingesetzt werden können. Weiterhin kann das Gestell 208 optional wiederum eine oder mehrere Aufnahmen 214 für einen oder mehrere, in den Figuren 10 und 11 nicht dargestellte Kleinteilkörbe 202 aufweisen. Bezüglich der möglichen Ausgestaltung dieser Kleinteilkörbe 202 kann exemplarisch auf die Figuren 5 und 6 verwiesen werden. Es können für die erste Halterung 194 und die dritte Halterung 276 dieselben Kleinteilkörbe 202 verwendet werden, da diese vorzugsweise lose in den Aufnahmen 214 aufgenommen sind.

Figur 11 zeigt die dritte Halterung 276 in einem gebrauchsfertigen Zustand, jedoch ohne Kleinteilkörbe 202. Der dritte Aufsatz 278 ist dabei auf den Grundkorb 178 aufgesetzt, wobei die Auflageecken 244 in den Positionierungshilfen 190 eingesetzt sind. Optional kann wiederum mindestens ein Verbindungselement 246 vorgesehen sein, beispielsweise wiederum zur Herstellung einer Rastverbindung. Bezüglich möglicher Ausgestaltungen dieses Verbindungselements 246 kann beispielsweise auf die obige Beschreibung der Figur 4 verwiesen werden.

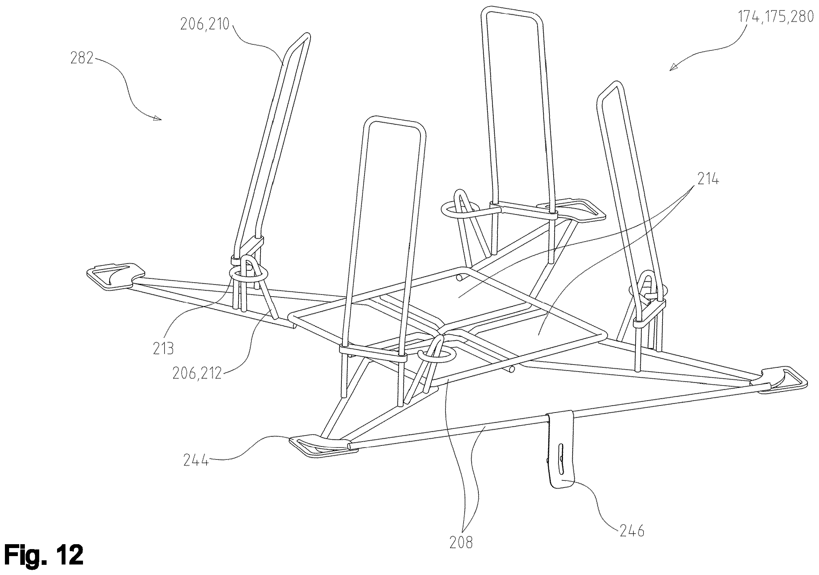

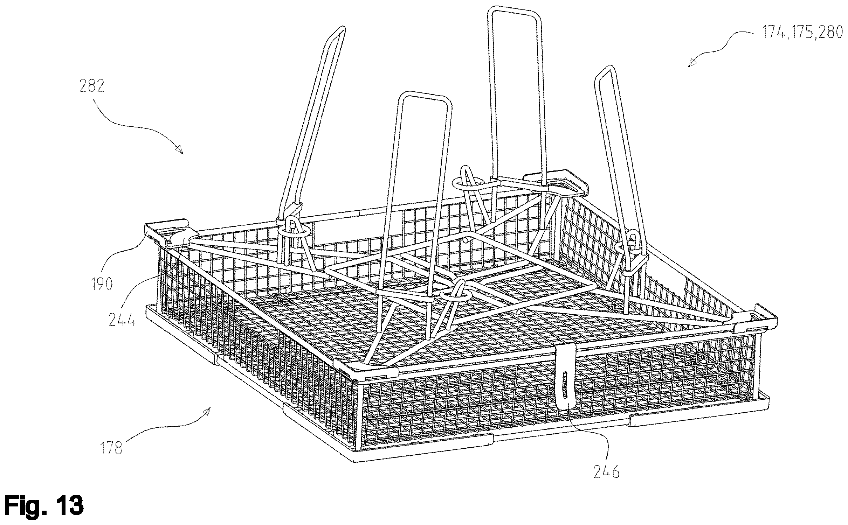

In den Figuren 12 und 13 ist exemplarisch ein Ausführungsbeispiel einer optionalen vierten Halterung 280 des Sortiments 175 dargestellt. Diese vierte Halterung 280 weist einen in Figur 12 im Detail dargestellten vierten Aufsatz 282 auf, welcher wiederum in einen Grundkorb 178 einsetzbar ist, wie in Figur 13 gezeigt ist. Diese vierte Halterung dient der Reinigung von Atemmasken 196, so dass diesbezüglich weitgehend auf die Beschreibung der Figuren 3 bis 6 oben verwiesen werden kann. Dementsprechend umfasst der vierte Aufsatz 282 der vierten Halterung 280 wiederum mindestens eine Masken-Haltevorrichtung 206. Im Falle der vierten Halterung 280 sind allerdings keine Lungenautomat-Haltevorrichtungen 216 vorgesehen.

Der vierte Aufsatz 282 weist wiederum, analog zu den Figuren 3 bis 6 oben, ein Gestell 208 auf, beispielsweise ein Drahtgestell. An diesem Gestell 208 sind Haltebügel 210 für Atemmasken 196 vorgesehen, sowie optional Haltebügel 212 für Innenmasken 204. Der vierte Aufsatz 282 kann wiederum optional, wie oben ausgeführt, einen oder mehrere Fixierelemente zum Fixieren der Atemmasken 196 und/oder der Innenmasken 204 aufweisen. So können beispielsweise wiederum ein oder mehrere Niederhalter 213 für die Innenmasken 204 vorgesehen sein. Diesbezüglich kann exemplarisch auf die obige Beschreibung verwiesen werden. Für weitere mögliche Ausgestaltungen kann ebenfalls auf die obige Beschreibung verwiesen werden. Exemplarisch sind in diesem Ausführungsbeispiel gemäß den Figuren 12 und 13 Masken-Haltevorrichtungen 206 für vier Atemmasken 196 sowie entsprechend vier Innenmasken 204 vorgesehen. Für das mögliche Aufbringen der Atemmasken 196 bzw. der Innenmaske 204 auf diese Masken-Haltevorrichtungen 206 kann exemplarisch auf die Darstellung gemäß Figur 6 verwiesen werden.

Optional können in dem Gestell 208 wiederum eine oder mehrere Aufnahmen 214 zum Aufnehmen eines oder mehrerer, in den Figuren 12 und 13 nicht dargestellter, Kleinteilkörbe 202 vorgesehen sein. Diese Kleinteilkörbe 202 können wiederum herausnehmbar in die Aufnahmen 214 eingestellt werden. Für die Ausgestaltung der Kleinteilkörbe 202 kann exemplarisch auf die obige Beschreibung verwiesen werden. Es können insbesondere auch dieselben Kleinteilkörbe 202 in der vierten Halterung 280 eingesetzt werden, welche auch beispielsweise in der ersten Halterung 194 verwendet werden.

Das Gestell 208 kann wiederum Auflageecken 244 aufweisen, welche in die Positionierungshilfen 190 des Grundkorbs 178 eingesetzt werden können. Dies ist in dem in Figur 13 gezeigten zusammengesetzten Zustand der vierten Halterung 280 erkennbar. Optional kann wiederum zusätzlich eine Verbindung durch ein oder mehrere Verbindungselemente 246 erfolgen, beispielsweise eine Rastverbindung. Diesbezüglich kann auf die obige Beschreibung der Figur 4 verwiesen werden.

### Bezugszeichenliste

- 110: Reinigungsvorrichtung
- 111: Reinigungssystem
- 112: Atemgeräte
- 114: Spülmaschine
- 116: Reinigungskammer
- 118: Spülkammer
- 120: Tür
- 122: Fluideinrichtung
- 124: Düsen
- 126: Waschdüsensystem
- 128: Nachspüldüsensystem
- 130: Waschleitungssystem
- 132: Reinigungsfluid, Waschfluid
- 134: Waschtank
- 136: Umwälzpumpe
- 138: Heizelement
- 140: Temperatursensor
- 142: Niveausensor
- 144: Ablaufleitung
- 146: Ablaufpumpe
- 148: Ablauf
- 150: Dosiersystem
- 152: Nachspülleitungssystem
- 154: Reinigungsfluid, Nachspülfluid
- 156: Nachspültank
- 158: Nachspültankheizung
- 160: Temperatursensor
- 162: Niveausensor
- 164: Zulauf
- 166: Ventil
- 168: Dosiersystem
- 170: Umkehrosmosevorrichtung
- 172: Drucksteigerungspumpe
- 174: Halterung
- 175: Sortiment
- 176: Steuerung
- 178: Grundkorb
- 180: Bodenteil
- 182: Seitenwände
- 184: Rand
- 186: Rahmen
- 188: Kantenschutz
- 190: Positionierungshilfen
- 192: Traggriffe
- 194: erste Halterung
- 196: Atemmasken
- 198: Lungenautomaten
- 200: erster Aufsatz
- 202: Kleinteilkorb
- 204: Innenmaske
- 206: Masken-Haltevorrichtung
- 208: Gestell
- 210: Haltebügel für Atemmaske
- 212: Haltebügel für Innenmaske
- 213: Niederhalter für Innenmaske
- 214: Aufnahmen
- 216: Lungenautomat-Haltevorrichtung
- 218: Reibbremsen
- 220: Druckbeaufschlagungsvorrichtung
- 222: Druckanschluss
- 224: Druckluftzufuhr
- 226: Schlauch
- 228: Druckgasleitung
- 230: Rahmen
- 232: Kupplung
- 234: Kappe
- 236: Kupplung
- 238: vorrichtungsseitige Druckbeaufschlagungsvorrichtung
- 240: Druckgasquelle
- 242: Ventil
- 244: Auflageecken
- 246: Verbindungselement
- 248: Deckel
- 250: Öffnung
- 252: Sichtscheibe
- 254: Innenseite
- 256: Halteband
- 258: Luftschlauch
- 260: zweite Halterung
- 262: zweiter Aufsatz
- 264: Rahmen
- 266: Traggestell
- 267: Traggestell-Haltevorrichtung
- 268: Stütze
- 270: ebene Platte
- 272: Querstreben
- 274: Tragriemen
- 276: dritte Halterung
- 278: dritter Aufsatz
- 280: vierte Halterung
- 282: vierter Aufsatz

## Patentansprüche

1. Sortiment (175) zur Reinigung von Atemgeräten (112), umfassend mindestens zwei Halterungen (174), wobei die Halterungen (174) umfassen:
- eine erste Halterung (194) zur Reinigung von Atemmasken (196) und Lungenautomaten (198), umfassend mindestens eine Masken-Haltevorrichtung (206) zur Positionierung mindestens einer Atemmaske (196), weiterhin umfassend mindestens eine Lungenautomat-Haltevorrichtung (216) zur Positionierung mindestens eines Lungenautomaten (198) und mindestens eine Druckbeaufschlagungsvorrichtung (220) mit mindestens einem Druckanschluss (222), wobei der Druckanschluss (222) mit dem Lungenautomaten (198) verbindbar ist und wobei die Druckbeaufschlagungsvorrichtung (220) eingerichtet ist, um den Lungenautomaten (198) während einer Reinigung mit Druckgas zu beaufschlagen, und
- mindestens eine zweite Halterung (260) zur Reinigung von Traggestellen (266) für Druckgasflaschen für Atemgeräte (112), umfassend mindestens eine Traggestell-Haltevorrichtung (267) zur Positionierung mindestens eines Traggestells (266), wobei die Halterungen (174) derart dimensioniert sind, dass diese austauschbar in eine Reinigungsvorrichtung (110) zur Reinigung der Atemgeräte (112) einbringbar sind, **dadurch gekennzeichnet, dass** die erste Halterung weiterhin mindestens einen Kleinteilkorb aufweist, wobei der Kleinteilkorb zur Aufnahme von Zubehörteilen während eines Reinigungsvorgangs eingerichtet ist,
wobei die Halterungen (174) jeweils einen Grundkorb (178) und jeweils mindestens einen Aufsatz (200, 262, 278, 282) aufweisen, wobei der Grundkorb (178) der Halterungen (174) baugleich ist und wobei sich die Aufsätze (200, 262, 278, 282) der Halterungen (174) unterscheiden.

2. Sortiment (175) nach dem vorhergehenden Anspruch, wobei die Masken-Haltevorrichtung (206) ein Gestell (208) umfasst, wobei das Gestell (208) mindestens einen Haltebügel (210) für die mindestens eine Atemmaske (196) aufweist, wobei die Atemmaske (196) auf den Haltebügel (210) aufgestülpt werden kann.

3. Sortiment (175) nach dem vorhergehenden Anspruch, wobei das Gestell (208) weiterhin mindestens einen weiteren Haltebügel (212) für mindestens eine Innenmaske (204) aufweist.

4. Sortiment (175) nach einem der vorhergehenden Ansprüche, wobei die Lungenautomat-Haltevorrichtung (216) mindestens eine Aufnahme aufweist, in welche ein Stutzen des Lungenautomaten (198) einsteckbar ist und in welcher der Stutzen nach dem Einstecken fest gehaltert ist.

5. Sortiment (175) nach einem der vorhergehenden Ansprüche, wobei die Druckbeaufschlagungsvorrichtung (220) mindestens eine Druckluftzufuhr (224) aufweist, wobei die Druckluftzufuhr (224) in eine Druckgasleitung (228) mündet, wobei der Druckanschluss (222) auf die Druckgasleitung (228) aufgesetzt ist, wobei die Druckgasleitung (228) als tragendes Bauteil ausgestaltet ist und zumindest die Lungenautomat-Haltevorrichtung (216) trägt.

6. Sortiment (175) nach einem der vorhergehenden Ansprüche, wobei die erste Halterung (194) eingerichtet ist, um eine Mehrzahl von Atemmasken (196) und eine Mehrzahl von Lungenautomaten (198) aufzunehmen und eine Mehrzahl von Masken-Haltevorrichtungen (206) und eine Mehrzahl von Lungenautomat-Haltevorrichtungen (216) sowie eine Mehrzahl von Druckanschlüssen (222) aufweist.

7. Sortiment (175) nach dem vorhergehenden Anspruch, wobei die Masken-Haltevorrichtungen (206) und die Lungenautomat-Haltevorrichtungen (216) alternierend entlang eines Umfangs der ersten Halterung (194) angeordnet sind.

8. Sortiment (175) nach einem der vorhergehenden Ansprüche, wobei eine Mehrzahl von Kleinteilkörben (202) vorgesehen ist, wobei die erste Halterung (194) zur Reinigung einer Mehrzahl von Atemmasken (196) und einer Mehrzahl von Lungenautomaten (198) eingerichtet ist, wobei jedem Paar bestehend aus einer Atemmaske (196) und einem Lungenautomaten (198) jeweils ein Kleinteilkorb (202) zugeordnet ist.

9. Sortiment (175) nach einem der vorhergehenden Ansprüche, wobei die Traggestell-Haltevorrichtung (267) mindestens eine nach oben von der zweiten Halterung (260) ragende Stütze (268) aufweist.

10. Sortiment (175) nach einem der vorhergehenden Ansprüche, wobei das Sortiment (175) weiterhin mindestens eine dritte Halterung (276) zur Reinigung von Lungenautomaten (198) aufweist, wobei die dritte Halterung (276) mindestens eine Lungenautomat-Haltevorrichtung (216) zur Positionierung mindestens eines Lungenautomaten (198) aufweist, wobei die dritte Halterung (276) weiterhin mindestens eine Druckbeaufschlagungsvorrichtung (220) mit mindestens einem Druckanschluss (222) aufweist, wobei der Druckanschluss (222) mit dem Lungenautomaten (198) verbindbar ist und wobei die Druckbeaufschlagungsvorrichtung (220) eingerichtet ist, um den Lungenautomaten (198) während einer Reinigung mit Druckgas zu beaufschlagen.

11. Sortiment (175) nach einem der vorhergehenden Ansprüche, wobei das Sortiment (175) weiterhin mindestens eine vierte Halterung (280) zur Reinigung von Atemmasken (196) aufweist, umfassend mindestens eine Masken-Haltevorrichtung (206) zur Positionierung mindestens einer Atemmaske (196).

12. Sortiment (175) nach einem der vorhergehenden Ansprüche, wobei die Aufsätze (200, 262, 278, 282) jeweils reversibel mit dem zugehörigen Grundkorb (178) verbindbar sind.

13. Reinigungssystem (111) zur Reinigung von Atemgeräten (112), umfassend:
- mindestens eine Reinigungsvorrichtung (110), umfassend mindestens eine Reinigungskammer (116) zur Aufnahme mindestens eines Atemgeräts (112), wobei die Reinigungsvorrichtung (110) weiterhin mindestens eine Fluideinrichtung (122) zur Beaufschlagung des Atemgeräts (112) mit mindestens einem Reinigungsfluid (132) aufweist, und
- mindestens ein Sortiment (175) nach einem der vorhergehenden Ansprüche, wobei die Halterungen (174) des Sortiments (175) wahlweise in der Reinigungskammer (116) aufnehmbar sind, wobei die Reinigungsvorrichtung (110) eingerichtet ist, um in mindestens einem Reinigungsschritt die erste Halterung (194) zu verwenden und um in diesem Reinigungsschritt über den Druckanschluss (222) mindestens einen in der ersten Halterung (194) aufgenommenen Lungenautomaten (198) mit Druckgas zu beaufschlagen.

14. Verfahren zur Reinigung von Atemgeräten (112), wobei das Reinigungssystem (111) nach dem vorhergehenden Anspruch verwendet wird, wobei das Verfahren mindestens einen ersten Reinigungsschritt und mindestens einen zweiten Reinigungsschritt umfasst,
- wobei in dem ersten Reinigungsschritt die erste Halterung (194) verwendet wird, wobei mindestens eine Atemmaske (196) in die Masken-Haltevorrichtung (206) der ersten Halterung (194) eingebracht wird, wobei mindestens ein Lungenautomat (198) in die Lungenautomat-Haltevorrichtung (216) eingebracht wird, wobei über den Druckanschluss (222) der Lungenautomat (198) mit Druckgas beaufschlagt wird und die Atemmaske (196) und der Lungenautomat (198) mittels der Fluideinrichtung (122) mit dem Reinigungsfluid (132) beaufschlagt werden,
- wobei in dem zweiten Reinigungsschritt die zweite Halterung (260) verwendet wird, wobei mindestens ein Traggestell (266) für Druckgasflaschen für Atemgeräte (112) in die Traggestell-Haltevorrichtung (267) eingebracht wird, wobei das Traggestell (266) mittels der Fluideinrichtung (122) mit dem Reinigungsfluid (132) beaufschlagt wird.

## Claims

1. Product range (175) for cleaning breathing apparatuses (112), including at least two holders (174), wherein the holders (174) include:
- a first holder (194) for cleaning breathing masks (196) and breathing valves (198), including at least one mask holding device (206) for positioning at least one breathing mask (196), additionally comprising at least one breathing valve holding device (216) for positioning at least one breathing valve (198) and at least one pressurizing device (220) with at least one pressure connection (222), wherein the pressure connection (222) is connectable to the breathing valve (198) and wherein the pressurizing device (220) is set up to apply pressurized gas to the breathing valves (198) during cleaning, and
- at least one second holder (260) for cleaning carrying frameworks (266) for pressurized gas cylinders for breathing apparatuses (112), including at least one carrying framework holding device (267) for positioning at least one carrying framework (266),
wherein the holders (174) are dimensioned in such a manner that they can be moved into a cleaning apparatus (110) for cleaning the breathing apparatuses (112) so as to be exchangeable, **characterized in that** the first holder additionally comprises at least one small parts basket, wherein the small parts basket is set up for receiving accessory parts during a cleaning operation, wherein the holders (174) comprise in each case a basic basket (178) and in each case at least one attachment (200, 262, 278, 282), wherein the basic basket (178) of the holders (174) is structurally the same and wherein the attachments (200, 262, 278, 282) of the holders (174) differ.

2. Product range (175) according to the preceding claim, wherein the mask holding device (206) includes a framework (208), wherein the framework (208) comprises at least one holding bracket (210) for the at least one breathing mask (196), wherein the breathing mask (196) can be put over the holding bracket (210).

3. Product range (175) according to the preceding claim, wherein the framework (208) additionally comprises at least one further holding bracket (212) for at least one inner mask (204).

4. Product range (175) according to any one of the preceding claims, wherein the breathing valve holding device (216) comprises at least one receiving means into which a connecting piece of the breathing valve (198) is pluggable and in which the connecting piece is held in a fixed manner once it has been plugged in.

5. Product range (175) according to any one of the preceding claims, wherein the pressurizing device (220) comprises at least one compressed air supply (224), wherein the compressed air supply (224) opens out into a pressurized gas line (228), wherein the pressure connection (222) is fitted onto the pressurized gas line (228), wherein the pressurized gas line (228) is developed as a carrying component and carries at least the breathing valve holding device (216).

6. Product range (175) according to any one of the preceding claims, wherein the first holder (194) is set up to receive a plurality of breathing masks (196) and a plurality of breathing valves (198) and comprises a plurality of mask holding devices (206) and a plurality of breathing valve holding devices (216) as well as a plurality of pressure connections (222) .

7. Product range (175) according to the preceding claim, wherein the mask holding devices (206) and the breathing valve holding devices (216) are arranged in an alternating manner along a periphery of the first holder (194).

8. Product range (175) according to any one of the preceding claims, wherein a plurality of small parts baskets (202) is provided, wherein the first holder (194) is set up for cleaning a plurality of breathing masks (196) and a plurality of breathing valves (198), wherein each pair consisting of one breathing mask (196) and one breathing valve (198) has assigned thereto in each case one small parts basket (202).

9. Product range (175) according to any one of the preceding claims, wherein the carrying framework holding device (267) comprises at least one support (268) which projects upward from the second holder (260) .

10. Product range (175) according to any one of the preceding claims, wherein the product range (175) additionally comprises at least one third holder (276) for cleaning breathing valves (198), wherein the third holder (276) comprises at least one breathing valve holding device (216) for positioning at least one breathing valve (198), wherein the third holder (276) additionally comprises at least one pressurizing device (220) with at least one pressure connection (222), wherein the pressure connection (222) is connectable to the breathing valve (198) and wherein the pressurizing device (220) is set up to apply pressurized gas to the breathing valves (198) during cleaning.

11. Product range (175) according to any one of the preceding claims, wherein the product range (175) additionally comprises at least one fourth holder (280) for cleaning breathing masks (196), comprising at least one mask holding device (206) for positioning at least one breathing mask (196).

12. Product range (175) according to any one of the preceding claims, wherein the attachments (200, 262, 278, 282) are in each case connectable in a reversible manner to the associated basic basket (178) .

13. Cleaning system (111) for cleaning breathing apparatuses (112), including:
- at least one cleaning apparatus (110), including at least one cleaning chamber (116) for receiving at least one breathing apparatus (112), wherein the cleaning apparatus (110) additionally comprises at least one fluid device (122) for applying at least one cleaning fluid (132) to the breathing apparatus (112), and
- at least one product range (175) according to any one of the preceding claims,
wherein the holders (174) of the product range (175) are receivable in a selective manner in the cleaning chamber (116), wherein said cleaning apparatus (110) is set up to use the first holder (194) in at least one cleaning step and to apply pressurized gas in said cleaning step by means of the pressure connection (222) to at least one breathing valve (198) received in the first holder (194).

14. Method for cleaning breathing apparatuses (112), wherein the cleaning system (111) according to the preceding claim is used, wherein the method includes at least one first cleaning step and at least one second cleaning step,
- wherein in the first cleaning step the first holder (194) is used, wherein at least one breathing mask (196) is moved into the mask holding device (206) of the first holder (194), wherein at least one breathing valve (198) is moved into the breathing valve holding device (216), wherein pressurized gas is applied to the breathing valve (198) by means of the pressure connection (222) and the cleaning fluid (132) is applied to the breathing mask (196) and the breathing valve (198) by means of the fluid device (122),
- wherein in the second cleaning step the second holder (260) is used, wherein at least one carrying framework (266) for pressurized gas cylinders for breathing apparatuses (112) is moved into the carrying framework holding device (267), wherein the cleaning fluid (132) is applied to the carrying framework (266) by means of the fluid device (122).

## Revendications

1. Assortiment (175) pour le nettoyage d'appareils respiratoires (112), comprenant au moins deux supports (174), les supports (174) comprenant :
- un premier support (194) pour le nettoyage de masques respiratoires (196) et de soupapes à la demande (198), comprenant au moins un dispositif de support de masque (206) pour le positionnement d'au moins un masque respiratoire (196), comprenant en outre au moins un dispositif de support de soupape à la demande (216) pour le positionnement d'au moins une soupape à la demande (198) et au moins un dispositif de sollicitation en pression (220) muni d'au moins un raccord de pression (222), le raccord de pression (222) pouvant être relié à la soupape à la demande (198) et le dispositif de sollicitation en pression (220) étant adapté pour solliciter la soupape à la demande (198) avec un gaz comprimé pendant un nettoyage, et
- au moins un deuxième support (260) pour le nettoyage de cadres porteurs (266) pour des bouteilles de gaz comprimé pour des appareils respiratoires (112), comprenant au moins un dispositif de support de cadre porteur (267) pour le positionnement d'au moins un cadre porteur (266),
les supports (174) étant dimensionnés de telle sorte que ceux-ci peuvent être introduits de manière interchangeable dans un dispositif de nettoyage (110) pour le nettoyage des appareils respiratoires (112), **caractérisé en ce que** le premier support présente en outre au moins un panier pour petites pièces, le panier pour petites pièces étant adapté pour la réception d'accessoires pendant une opération de nettoyage,
les supports (174) présentant chacun un panier de base (178) et chacun au moins un élément rapporté (200, 262, 278, 282), le panier de base (178) des supports (174) étant de construction identique et les éléments rapportés (200, 262, 278, 282) des supports (174) étant différents.

2. Assortiment (175) selon la revendication précédente, le dispositif de support de masque (206) comprenant un cadre (208), le cadre (208) présentant au moins un étrier de support (210) pour l'au moins un masque respiratoire (196), le masque respiratoire (196) pouvant être monté sur l'étrier de support (210).

3. Assortiment (175) selon la revendication précédente, le cadre (208) présentant en outre au moins un autre étrier de support (212) pour au moins un masque intérieur (204).

4. Assortiment (175) selon l'une quelconque des revendications précédentes, le dispositif de support de soupape à la demande (216) présentant au moins un logement dans lequel un embout de la soupape à la demande (198) peut être inséré et dans lequel l'embout est maintenu fermement après l'insertion.

5. Assortiment (175) selon l'une quelconque des revendications précédentes, le dispositif de sollicitation en pression (220) présentant au moins une alimentation en air comprimé (224), l'alimentation en air comprimé (224) débouchant dans une conduite de gaz comprimé (228), le raccord de pression (222) étant placé sur la conduite de gaz comprimé (228), la conduite de gaz comprimé (228) étant conçue sous forme de composant porteur et portant au moins le dispositif de support de soupape à la demande (216).

6. Assortiment (175) selon l'une quelconque des revendications précédentes, le premier support (194) étant adapté pour recevoir une pluralité de masques respiratoires (196) et une pluralité de soupapes à la demande (198) et présentant une pluralité de dispositifs de support de masque (206) et une pluralité de dispositifs de support de soupape à la demande (216) ainsi qu'une pluralité de raccords de pression (222).

7. Assortiment (175) selon la revendication précédente, les dispositifs de support de masque (206) et les dispositifs de support de soupape à la demande (216) étant agencés de manière alternée le long d'une circonférence du premier support (194).

8. Assortiment (175) selon l'une quelconque des revendications précédentes, une pluralité de paniers pour petites pièces (202) étant prévue, le premier support (194) étant adapté pour le nettoyage d'une pluralité de masques respiratoires (196) et d'une pluralité de soupapes à la demande (198), un panier pour petites pièces (202) étant associé respectivement à chaque paire constituée d'un masque respiratoire (196) et d'une soupape à la demande (198).

9. Assortiment (175) selon l'une quelconque des revendications précédentes, le dispositif de support de cadre porteur (267) présentant au moins un appui (268) qui s'étend vers le haut à partir du deuxième support (260) .

10. Assortiment (175) selon l'une quelconque des revendications précédentes, l'assortiment (175) présentant en outre au moins un troisième support (276) pour le nettoyage de soupapes à la demande (198), le troisième support (276) présentant au moins un dispositif de support de soupape à la demande (216) pour le positionnement d'au moins une soupape à la demande (198), le troisième support (276) présentant en outre au moins un dispositif de sollicitation en pression (220) muni d'au moins un raccord de pression (222), le raccord de pression (222) pouvant être relié à la soupape à la demande (198) et le dispositif de sollicitation en pression (220) étant adapté pour solliciter la soupape à la demande (198) avec un gaz comprimé pendant un nettoyage.

11. Assortiment (175) selon l'une quelconque des revendications précédentes, l'assortiment (175) présentant en outre au moins un quatrième support (280) pour le nettoyage de masques respiratoires (196), comprenant au moins un dispositif de support de masque (206) pour le positionnement d'au moins un masque respiratoire (196).

12. Assortiment (175) selon l'une quelconque des revendications précédentes, les éléments rapportés (200, 262, 278, 282) pouvant chacun être reliés de manière réversible au panier de base (178) correspondant.

13. Système de nettoyage (111) pour le nettoyage d'appareils respiratoires (112), comprenant :
- au moins un dispositif de nettoyage (110), comprenant au moins une chambre de nettoyage (116) pour la réception d'au moins un appareil respiratoire (112), le dispositif de nettoyage (110) présentant en outre au moins un appareil à fluide (122) pour solliciter l'appareil respiratoire (112) avec au moins un fluide de nettoyage (132), et
- au moins un assortiment (175) selon l'une quelconque des revendications précédentes,
les supports (174) de l'assortiment (175) pouvant être sélectivement reçus dans la chambre de nettoyage (116), le dispositif de nettoyage (110) étant adapté pour utiliser le premier support (194) dans au moins une étape de nettoyage et pour solliciter avec du gaz comprimé, par l'intermédiaire du raccord de pression (222), au moins une soupape à la demande (198) reçue dans le premier support (194) dans cette étape de nettoyage.

14. Procédé de nettoyage d'appareils respiratoires (112), le système de nettoyage (111) selon la revendication précédente étant utilisé, le procédé comprenant au moins une première étape de nettoyage et au moins une deuxième étape de nettoyage,
- dans la première étape de nettoyage, le premier support (194) étant utilisé, au moins un masque respiratoire (196) étant introduit dans le dispositif de support de masque (206) du premier support (194), au moins une soupape à la demande (198) étant introduite dans le dispositif de support de soupape à la demande (216), la soupape à la demande (198) étant sollicitée avec du gaz comprimé par l'intermédiaire du raccord de pression (222) et le masque respiratoire (196) et la soupape à la demande (198) étant sollicités avec le fluide de nettoyage (132) au moyen de l'appareil à fluide (122),
- dans la deuxième étape de nettoyage, le deuxième support (260) étant utilisé, au moins un cadre porteur (266) pour des bouteilles de gaz comprimé pour des appareils respiratoires (112) étant introduit dans le dispositif de support de cadre porteur (267), le cadre porteur (266) étant sollicité avec le fluide de nettoyage (132) au moyen de l'appareil à fluide (122).
